# EUROPÄISCHE PATENTSCHRIFT

(11) **EP 1 864 964 B1**
(45) Veröffentlichungstag und Bekanntmachung des Hinweises auf die Patenterteilung: **17.11.2010**
(21) Anmeldenummer: 07107103.9
(22) Anmeldetag: 27.04.2007
(51) Int. Cl.: C07C 69/67, C07C 69/704, C08K 5/00, C08K 5/10, C08K 5/11

(54) **Tripentylcitrate und deren Verwendung als Weichmacher**
Tripentyl citrates and their use as plasticisers
Les citrates de tripentyle et leur utilisation comme plastifiants

(30) Priorität: 08.06.2006 DE 102006026624
(43) Veröffentlichungstag der Anmeldung: 12.12.2007
(73) Patentinhaber: Evonik Oxeno GmbH, 45772 Marl (DE)
(72) Erfinder: Graß, Michael Dr., 45721, Haltern am See (DE); Woelk-Faehrmann, Michael, 45768, Marl (DE)

(56) Entgegenhaltungen:
- EP-A1- 1 063 257
- WO-A-03/008369
- WO-A-2005/028407
- DE-A1- 3 520 750
- DE-A1- 19 842 370
- US-A1- 2005 198 894
- SALIT N I ET AL: "Esters of Citric and malic Acids" JOURNAL OF GENERAL CHEMISTRY OF THE USSR, CONSULTANTS BUREAU, NEW YORK, NY, US, Bd. 33, Nr. 8, 1963, Seiten 2674-2676, XP008080925 ISSN: 0022-1279
- DATABASE CA [Online] CHEMICAL ABSTRACTS SERVICE, COLUMBUS, OHIO, US; SALIT, N. I. ET AL: "Monomers based on the compounds of methacrylic acid with dialkyl malates and trialkyl citrates and their polymerization" XP002443488 gefunden im STN Database accession no. 1965:403591 & NAUCHN. TR., TASHKENTSK. GOS. UNIV. , NO. 263, 122-6, 1964,
- FRANK ALBER, DIRK KLOPPENBURG, HANS KLOPPENBURG, DIRK SCHÄR, RAINER UTZ, GEORG SCHUPFNER: "Citric acid esters based on mixtures of alcohols-New kind of polymer additives and plasticizers for a variety of polymers" IP.COM PRIOR ART DATABASE, [Online] 13. Juli 2004 (2004-07-13), XP002443474 Gefunden im Internet: URL:www.ip.com/pubview/IPCOM000029795D> [gefunden am 2007-07-04]

## Beschreibung

Die vorliegende Erfindung betrifft Tripentylcitrate mit acylierter, bevorzugt acetylierter OH-Gruppe, ein Verfahren zu deren Herstellung sowie die Verwendung dieser Verbindungen als Weichmacher für Kunststoffe.

Polyvinylchlorid (PVC) gehört zu den wirtschaftlich bedeutendsten Polymeren. Es findet sowohl als Hart-PVC als auch als Weich-PVC vielfältige Anwendungen.

Zur Erzeugung eines Weich-PVC werden dem PVC Weichmacher zugesetzt, wobei in der überwiegenden Anzahl der Fälle Phthalsäureester, insbesondere Di-2-ethylhexylphthalat (DEHP), Diisononylphthalat (DINP) und Diisodecylphthalat (DIDP) Verwendung finden.

Auf Grund von Diskussionen um reproduktionstoxische Effekte, die bereits in einigen Fällen zu einer verstärkten gefahrstoffrechtlichen Kennzeichnung und im Falle von Kleinkinderspielzeug auch zu Verwendungsbeschränkungen geführt haben, muss davon ausgegangen werden, dass die Verwendung dieser Phthalate künftig, insbesondere in sensiblen Anwendungen wie Lebensmittelverpackungen und Medizinalanwendungen deutlich zurückgehen wird. Es besteht daher Bedarf nach nicht kennzeichnungspflichtigen Weichmachern, die beispielsweise als DEHP-Ersatz Verwendung finden können und die aus Rohstoffen hergestellt werden, die weltweit in großen Mengen verfügbar sind.

Eine denkbare Alternative ist die Verwendung von Weichmachern auf Basis von Citronensäure. Insbesondere der prominenteste Vertreter dieser Verbindungsklasse, das Acetyl-tri-n-butyl-citrat (ATBC) wird nicht zuletzt seit der o. g. Verwendungsbeschränkung für gewisse Phthalate verstärkt zur Herstellung von Kinderspielzeug aus Weich-PVC eingesetzt. Unterstützt wird dies auch durch eine Stellungnahme des früheren toxikologischen Expertengremiums der EU, dem Scientific Committee for Toxicology, Ecology and Ecotoxicology (CSTEE), wonach die Verwendung dieses Weichmachers in Weich-PVC-Spielzeug auch für Kleinkinder kein Risiko beinhaltet.

Es ist jedoch bekannt, dass ATBC eine höhere Flüchtigkeit und Migrationsrate als beispielsweise DEHP aufweist und somit noch Optinierungspotential besteht. Es hat daher nicht an Versuchen gefehlt, strukturell variierte Citronensäureester zu entwickeln, die diese Nachteile nicht mehr zeigen. Prinzipiell kann dies durch Verwendung längerkettiger Alkohole zur Veresterung erreicht werden. Lange bekannt und auch im Markt eingeführt sind daher beispielsweise Acetyl-tri-2-ethylhexylcitrat (ATEHC) oder Butyryl-tri-n-hexylcitrat (BTHC). Die Herstellung von ATBC wird z. B. in WO 03/008369 beschrieben, auf deren Inhalt in dieser Anmeldung Bezug genommen wird.

Daneben sind auch Citratester mit freier, d. h. nicht acylierter OH-Gruppe beschrieben. Beispielsweise werden in EP 1 063 257 Citronensäuretrialkylester mit Alkyl = C₆ bis C₁₀, erwähnt, wobei die Alkylketten bevorzugt linear sind. Diese zeichnen sich im Vergleich zu ihren carboxylierten Analoga in der Regel durch eine verbesserte Effizienz und Gelierung, allerdings aber auch durch eine schlechtere thermische Stabilität aus.

Neben den Estern der Citronensäure bzw. acetylierter Citroensäure mit nur einem Alkohol wie beispielsweise Butanol (also ATBC) oder 2-Ethylhexanol (also ATEHC) sind auch Ester auf Basis von Alkoholgemischen mit unterschiedlichen C-Zahlen bekannt. Schär et al. beschreiben in Ip.com Journal (2004), 4 (8), S. 15 ff die Verwendung von Citronensäureestern (mit freier oder derivatisierter OH-Gruppe) auf Basis von Alkoholmischungen, die aus mindestens zwei verschiedenen Alkoholen im Bereich von C₂ bis C₂₂ bestehen und bei denen die Alkohole vor der Veresterung gezielt gemischt werden.

In EP 1 256 566 werden Mischungen von Citronensäureestern beschrieben, deren Alkylketten zu einem bestimmten Prozentsatz aus Butyl und zu einem komplementären Prozentsatz aus längeren Resten bestehen.

DE 3520750 beschreibt Verfahren zur Herstellung von Citratestern und deren Verwendung für medizinische Artikel.

Citronensäureester auf Basis linearer Alkohole (C₆ und höher) sind allerdings in der Regel relativ teuer, da die Alkohole durch Ethylenoligomerisierung oder über Fettsäurehydrierung bzw. Hydrierung der Fettsäureester, wie z. B. Methylester, hergestellt werden müssen. Eine großtechnische Herstellung im Maßstab von vielen Tausend Tonnen setzt jedoch die Verwendung preislich wettbewerbsfähiger Alkohole voraus. Längerkettige Ester beispielsweise mit C₈-Alkoholen sind sehr gering flüchtig, weisen allerdings eine für bestimmte Plastisolverarbeitungs-Techniken zu langsame Gelierung auf. Die mit der geringeren Effizienz einher gehende notwendige Höherdosierung trägt in der Regel zur weiteren Rezepturverteuerung und zu höherem Rohstoffverbrauch bei.

Es bestand daher die Aufgabe, neue alternative Weichmacher, vorzugsweise alternative Citronensäureester zu finden, die vorzugsweise gute Verarbeitungseigenschaften zeigen, eine gute weichmachende Wirkung (Effizienz) aufweisen, nur wenig flüchtig sind und/oder deren Alkohole leicht in großen Mengen günstig herstellbar sind.

Überraschenderweise wurde gefunden, dass Citronensäurepentylester, insbesondere solche mit acetylierter OH-Gruppe, eine oder mehrere dieser Anforderungen erfüllen.

Gegenstand der vorliegenden Erfindung sind deshalb Citronensäureester der Formel I, 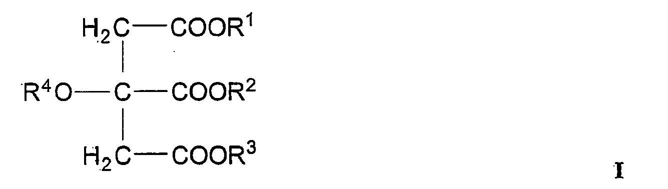 welche dadurch gekennzeichnet sind, dass die Reste R¹, R² und R³ jeweils ein Alkylrest mit einer Anzahl an Kohlenstoffatomen von 5 sind und der Rest R⁴ ein Carbonsäurerest gemäß Anspruch 1 ist sowie ein Verfahren zur Herstellung von Citronensäureestern der Formel I, welches dadurch gekennzeichnet ist, dass Citronensäure oder ein Citronensäurederivat mit einem Alkohol umgesetzt wird, der 5 Kohlenstoffatome aufweist.

Ebenso ist Gegenstand der vorliegenden Erfindung die Verwendung der erfindungsgemäßen Citronensäureester als Weichmacher.

Außerdem ist Gegenstand der vorliegenden Erfindung eine Zusammensetzung enthaltend einen erfindungsgemäßen Citronensäureester sowie die Verwendung einer solchen Zusammensetzung bzw. Weichmacherzusammensetzung in Kunststoffzusammensetzungen, Klebstoffen, Dichtungsmassen, Lacken, Farben, Plastisolen oder Tinten. Aus den erfindungsgemäßen Weichmachern hergestellte Kunststoffprodukte können beispielsweise sein: Profile, Dichtungen, Lebensmittelverpackungen, Folien, Spielzeug, Medizinalartikel, Dachbahnen, Kunstleder, Fußbodenbeläge, Unterbodenschutz, beschichtete Geweben, Tapeten, Kabel und Drahtummantelungen.

Die erfindungsgemäßen Citronensäureester haben den Vorteil, dass sie als sogenannte Primärweichmacher eingesetzt werden können. Unter einem Primärweichmacher wird üblicherweise und im Rahmen der vorliegenden Erfindung ein Weichmacher verstanden, der mit dem entsprechenden Polymeren in weiten Konzentrationsbereichen verträglich ist. Im Vergleich mit ATBC weisen die erfindungsgemäßen Citronensäureester bei vergleichbarer Effizienz, ausgedrückt über die Shore Härte A, eine deutlich geringere Flüchtigkeit auf.

Als Weichmacher können die erfindungsgemäßen Citronensäureester bestimmte Phthalate, wie z. B. Di-2-ethyl-hexylphthalat (DEHP) ersetzen. Im Vergleich zu DEHP, dem weltweit nach wie vor wichtigsten PVC-Weichmacher, liegen Flüchtigkeit und Gelierfahigkeit der erfindungsgemäßen Citrate auf einem ähnlichen Niveau. Vorteile ergeben sich in der Plastisolverarbeitung durch die niedrigere Plastisolviskosität, auch nach längerer Lagerung. Im Vergleich zu DINP ist die weichmachende Wirkung (Effizienz) der Pentylcitrate und Acetylpentylcitrate stets höher oder im Grenzfall des reinen Acetyl-tris-(3-methylbutyl)citrat zumindest gleich.

Da für Acetylpentylcitrate ähnlich gute toxikologische Eigenschaften wie für das ATBC zu erwarten sind, könnten diese als Ersatz für Phthalate insbesondere in kritischen Anwendungen wie Kinderspielzeugen oder Lebensmittelverpackungen verwendet werden. Die erfindungsgemäßen Acetylpentylcitrate haben weiterhin den Vorteil, dass sie auf einem Alkohol basieren, der auf einfache Weise hergestellt werden kann und dessen Rohstoff Crack C₄ oder Raffinat in großen Mengen zur Verfügung steht.

Nachfolgend wird die Erfindung beispielhaft beschrieben, ohne dass die Erfindung, deren Schutzbereich sich aus den Ansprüchen und der Beschreibung ergibt, darauf beschränkt sein soll. Auch die Ansprüche selbst gehören zum Offenbarungsgehalt der vorliegenden Erfindung. Sind nachfolgend Bereiche, allgemeine Formeln oder Verbindungsklassen angegeben, so soll die Offenbarung nicht nur die entsprechenden Bereiche oder Gruppen von Verbindungen erfassen, die explizit erwähnt sind, sondern auch alle Teilbereiche und Teilgruppen von Verbindungen, die durch Weglassen von einzelnen Werten (Bereichen) oder Verbindungen erhalten werden können, ohne dass diese aus Gründen der besseren Übersichtlichkeit explizit genannt worden sind.

Die erfindungsgemäßen Citronensäureester der Formel I, 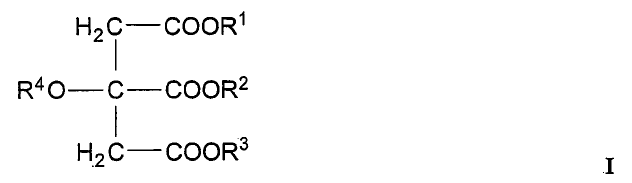 zeichnen sich dadurch aus, dass die Reste R¹, R² und R³ jeweils ein Alkylrest mit einer Anzahl an Kohlenstoffatomen von 5 sind und der Rest R⁴ ein Ameisensäurerest, ein Essigsäurerest, ein Propionsäurerest, ein Buttersäurerest oder ein Valerionsäureres ist. Besonders bevorzugt ist der Rest R⁴ ein Acetylrest. Carboxylierte Pentylcitrate weisen gegenüber den Pentylcitraten mit freier OH-Gruppe den Vorteil auf, dass sie thermisch deutlich stabiler sind, was sich beispielsweise durch eine verlangsamte Braunfärbung (geringere Stabilisierung reicht dann aus) von mit ihnen weichgemachten PVC-Folien zeigt. Ein weitere Vorteil der carboxylierten Pentylcitrate besteht darin, dass der Viskositätsanstieg in Plastisolen mit der Zeit insbesondere bei Verwendung der acetylierten Ester deutlich geringer ist als bei nicht carboxylierten Pentylcitraten, weshalb Plastisole auf Basis von erfindungsgemäßen carboxylierten Pentylcitraten eine bessere Lagerstabilität aufweisen.

Die Alkylreste können in einem Estermolekül gleich oder unterschiedlich sein. Werden bei der Herstellung der Ester keine isomeren-reinen Alkohole eingesetzt, werden üblicherweise Citronensäuretrialkylestergemische erhalten, die Estermoleküle enthalten, die unterschiedliche Alkylreste aufweisen.

Es kann vorteilhaft sein, wenn die Alkylreste R¹, R² und R³ eine längste Kohlenstoffkette von zumindest 4 Kohlenstoffatomen aufweisen und eine Gesamtzahl an Kohlenstoffatomen pro Alkylrest von 5 aufweisen. Bevorzugt sind die Alkylreste R¹, R² und R³ zu mehr als 60 % n-Pentyl-Reste. Der Anteil der Pentyl-Reste bezieht sich dabei auf den Mittelwert aller in den Citronensäureestern enthaltenen Alkylreste. Bevorzugt sind die Alkylreste der Citronensäureester von 70 bis 99,9 % n-Pentyl-Reste und von 30 bis 0,1 % Methylbutyl-Reste, insbesondere 2-Methylbutylreste, besonders bevorzugt von 85 bis 98 % n-Pentyl-Reste und von 15 bis 2 % Methylbutyl-Reste, insbesondere 2-Methylbutylreste, und ganz besonders bevorzugt von 90 bis 96 % n-Pentyl-Reste und von 10 bis 4 % Methylbutyl-Reste, insbesondere 2-Methylbutylreste. Vorzugsweise sind die Methylbutyl-Reste zu mehr als 50 %, bevorzugt zu mehr als 75 % und besonders bevorzugt zu mehr als 95 % 2-Methylbutyl-Reste. Je nach Rohstoffverfügbarkeit und Verwendungszweck des entsprechenden Weichmachers kann es aber auch vorteilhaft sein, wenn mindestens 40 %, vorzugsweise 40 bis 100 %, besonders bevorzugt 50 bis 99 % der C₅-Alkyl-Reste der Citronensäureester 3-Methylbutylreste sind (Angaben jeweils in Mol-%).

Die prozentuale Verteilung der C₅-Alkyl-Reste kann auf einfache Weise durch Verseifen der Ester, Abtrennen des erhaltenen Alkohols und gaschromatographische (GC) Analyse des erhaltenen Alkohols erfolgen. Beispielsweise kann die gaschromatographische Trennung auf einer Polydimethylsiloxan-Säule (z. B. DB 5) als stationärer Phase mit einer Länge von 60 m, einem inneren Durchmesser von 0,25 mm und einer Filmdicke von 0,25 µm durchgeführt werden. Alternativ kann diese Information auch über NMR-Spektroskopie erhalten werden.

Die erfindungsgemäßen Citronensäureester können z. B. mit dem erfindungsgemäßen Verfahren hergestellt werden. Dieses Verfahren zur Herstellung von Citronensäureestern der Formel I 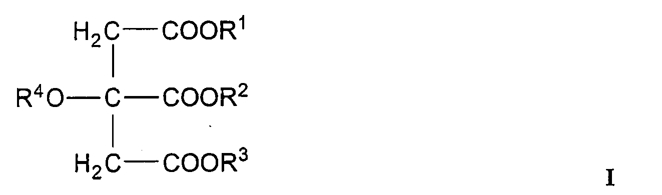 bei dem die Reste R¹, R² und R³ jeweils ein Alkylrest mit einer Anzahl an Kohlenstoffatomen von 5 sind und der Rest R⁴ ein Carbonsäurerest gemäß Anspruch 1 ist, zeichnet sich dadurch aus, dass Citronensäure oder ein Citronensäurederivat mit einem Alkohol umgesetzt wird, der 5 Kohlenstoffatome aufweist. Vorzugsweise erfolgt die Umsetzung der Citronensäure mit den Pentanolen bei einer Temperatur von größer 120°C, bevorzugt zwischen 120 und 160 °C.

Die Citronensäureester können aus den entsprechenden Alkoholen oder Alkoholgemischen durch Umsetzung mit Citronensäure oder deren Derivaten hergestellt werden. Insbesondere können die Citronensäureester bevorzugt durch Veresterung mit Citronensäure, z. B. mit Citronensäure-Monohydrat oder -Anhydrat, wie Citronensäure häufig kommerziell erhältlich ist, oder auch durch Umesterung aus Citronensäureestern mit kürzeren Alkoholresten hergestellt werden. Verschiedene Verfahren zur Herstellung von Trialkylcitraten und Carboxyltrialkylcitraten sind bekannt und teilweise weiter oben zitiert. Diese können auch zur Herstellung der erfindungsgemäßen Tripentylcitrate verwendet werden. Daher wird auf diese Publikationen ausdrücklich Bezug genommen. Im Falle der Umesterung kann als Ausgangsstoff z. B. Trimethylcitrat oder Triethylcitrat eingesetzt werden.

Alkohole zur Herstellung der erfindungsgemäßen Citronensäureester können alle gesättigten Alkohole sein, die aus 5 Kohlenstoffatomen bestehen und eine OH-Gruppe aufweisen. Vorzugsweise werden nicht-cyclische Alkohole eingesetzt, die eine längste Kohlenstoffkette von zumindest 4 Kohlenstoffatomen aufweisen und eine Gesamtzahl an Kohlenstoffatomen pro Alkylrest von 5 aufweisen. Hierbei sind primäre Alkohole besonders bevorzugt. Beispielhaft seien hier n-Pentanol, 2-Methylbutanol und 3-Methylbutanol genannt sowie Gemische dieser Alkohole.

Vorzugsweise werden in dem erfindungsgemäßen Verfahren Alkoholgemische eingesetzt, die zu mehr als 60 Massen-% n-Pentanol aufweisen. Bevorzugt werden Gemische aus n-Pentanol und 2-Methylbutanol im Massen-Verhältnis von 99,9 bis 70 % n-Pentanol und 0,1 bis 30 % Methylbutanol, insbesondere 2-Methylbutanol, besonders bevorzugt von 98 bis 85 % n-Pentanol und von 2 bis 15 % Methylbutanol, insbesondere 2-Methylbutanol eingesetzt. Wie bereits oben ausgeführt kann es je nach Rohstoffverfügbarkeit und Verwendungszweck des entsprechenden Weichmachers aber auch vorteilhaft sein, wenn Alkoholgemische eingesetzt werden, die mindestens 40 Massen-%, vorzugsweise 40 bis 100 Massen-%, besonders bevorzugt 50 bis 99 Massen-% 3-Methylbutanol aufweisen.

Bevorzugt können in dem erfindungsgemäßen Verfahren zur Herstellung von Citronensäureestern der Formel I primäre Alkohole oder Alkoholgemische wie sie beispielsweise durch Hydroformylierung eines Alkens mit nachfolgender Hydrierung erhalten werden können, eingesetzt werden. So kann zum Beispiel n-Pentanol durch Hydroformylierung von 1-Buten und nachfolgender Hydrierung des Valeraldehyds zu n-Pentanol hergestellt werden.

Vorstufen für Pentanole sind vorzugsweise technische Kohlenwasserstoffgemische, die ein oder mehrere Olefin(e) mit 4 Kohlenstoffatomen enthalten. Die bedeutendste Quelle für C₄-Olefine ist der C₄-Schnitt des Crackbenzins aus Steamcrackern. Daraus wird nach Extraktion(sdestillation) des Butadiens oder dessen Selektivhydrierung zu einem n-Butengemisch ein Kohlenwasserstoffgemisch (Raffinat I oder hydriertes Crack-C₄) hergestellt, das Isobuten, 1-Buten und die beiden 2-Butene (cis und trans) enthält. Ein anderer Rohstoff für C₄-Olefine ist der C₄-Schnitt aus FCC-Anlagen, der wie oben beschrieben aufgearbeitet werden kann. C₄-Olefine, hergestellt durch Fischer-Tropsch-Synthese, sind nach Selektivhydrierung des darin enthaltenen Butadiens zu n-Butenen ebenfalls ein geeigneter Einsatzstoff. Darüber hinaus können Olefingemische, die durch Dehydrierung von C₄-Kohlenwasserstoffen oder durch Metathesereaktionen erhalten werden, oder andere technische Olefinströme geeignete Einsatzstoffe sein. Neben dem Raffinat I sind als Vorstufen für die Pentanole auch Raffinat II, Raffinat III, ein Strom welcher durch Abtrennung des größten Teils an 1-Buten aus dem Raffinat II erhalten wird, und sogenanntes Rohbutan, welches nach einer Oligomerisierung von Raffinat II anfällt und welches neben Alkanen als Olefin ausschließlich geringe Mengen von 2-Buten aufweist, geeignet. Der Vorteil der Verwendung von Raffinat II, Raffinat III oder Rohbutan als Vorstufe für Pentanole liegt darin, dass diese Vorstufen kein bzw. nahezu kein Isobuten aufweisen, weshalb die erhaltenen Pentanole kein bzw. nur geringe Mengen (kleiner 0,5 Massen-% in Bezug auf die Pentanole) an 3-Methylbutanol aufweisen. Sollen die C₅-Alkohole größere Anteile an 3-Methylbutanol aufweisen, kann reines Isobuten, wie es z.B. durch Spaltung von Methyl-tert.-butylether oder tert.-Butanol erhalten werden kann, oder direkt Crack-C₄ oder Raffinat I eingesetzt werden.

Wegen des häufig sehr hohen Trennaufwands zur Trennung der Einsatzgemische kann es vorteilhaft sein, die im als Einsatzgemisch einzusetzenden technischen Gemisch vorliegenden Olefine nicht zu trennen, sondern die Gemische direkt einzusetzen.

Besonders bevorzugt wird in dem erfindungsgemäßen Verfahren Alkohol eingesetzt, der durch ein Verfahren umfassend die Schritte
a) Hydroformylierung von C₄-Olefinen unter Erhalt von C₅- Aldehyden und
b) Hydrierung der in Schritt a) erhaltenen Aldehyde zu den entsprechenden Alkoholen erhalten wird. Die Schritte a) und b) können dabei auch zeitgleich in einem Reaktor durchgeführt werden.

Es kann vorteilhaft sein, wenn nach Verfahrensschritt a) (Hydroformylierung) und/oder b) (Hydrierung) eine Auftrennung der in diesen Verfahrensstufen erhaltenen Produktgemische in die einzelnen Isomeren erfolgt. Eine solche Auftrennung kann z. B. thermisch, insbesondere durch Destillation erfolgen.

### Verfahrensschritt a)

Die Hydroformylierung aller Olefine im Einsatzgemisch kann in einer Stufe erfolgen. Dies kann insbesondere dann vorteilhaft sein, wenn bei der Hydroformylierung nur eine olefinische Verbindung im Einsatzgemisch vorhanden ist. Die Hydroformylierung von z. B. ausschließlich 1-Buten oder Isobuten als Olefin enthaltenden Einsatzgemischen kann in einer Stufe unter den unten für die erste Stufe beschriebenen Bedingungen und mit dem dort beschriebenen Katalysator durchgeführt werden. Die Hydroformylierung von z. B. ausschließlich 2-Buten als Olefin enthaltenden Einsatzgemischen kann in einer Stufe unter den unten für die zweite Stufe beschriebenen Bedingungen und mit dem dort beschriebenen Katalysator durchgeführt werden.

Da die isomerenreinen Olefine als Einsatzgemische aber häufig nicht vorliegen, sondern meistens technische Gemische von C₄-Kohlenwasserstoffen, wie sie oben beschrieben wurden, als Einsatzgemische vorliegen, wird im erfindungsgemäßen Verfahren in Verfahrensschritt a) vorzugsweise ein Gemisch von Olefinen eingesetzt, welches Isobuten und/oder 1-Buten und 2-Butene aufweist.

Die Hydroformylierung der im Einsatzgemisch enthaltenen Olefine kann wiederum in einer Stufe erfolgen. Dazu wird vorzugsweise ein Katalysator eingesetzt, der vermag, Olefine mit unterschiedlicher Lage der Doppelbindung und/oder Anzahl der Verzweigungen zu hydroformylieren. Katalysatoren, die dafür geeignet sind, bewirken aber meistens nur eine geringe Selektivität für die Bildung von Produkten (Aldehyde, Alkohole, Formiate), die durch endständige Hydroformylierung entstanden sind, und/oder zeigen eine zu geringe Reaktionsgeschwindigkeit für ein technisches Verfahren.

Wenn aus dem Hydroformylierungsprodukten Einsatzalkohole, insbesondere Pentanole bzw. Pentanolgemische mit möglichst geringem Verzweigungsgrad gewonnen werden sollen, ist es zweckmäßig, die Hydroformylierung derart durchzuführen, dass ein hoher Anteil an Produkten, die durch endständige Hydroformylierung entstanden sind, gewonnen wird; denn nur die endständig hydroformylierten Produkte weisen den gleichen Verzweigungsgrad wie ihre Ausgangsolefine auf, bei mittelständiger Hydroformylierung dagegen erhöht sich der Verzweigungsgrad des entstandenen Produkts um 1, was in vielen Fällen zu einer Verschlechterung der anwendungstechnischen Eigenschaften der hieraus hergestellten Folgeprodukte führen kann.

Die in einem technischen Gemisch vorliegenden Olefine unterscheiden sich in ihrer Reaktivität bei der Hydroformylierung beträchtlich. Im Allgemeinen sind Olefine mit endständigen Doppelbindungen reaktiver als Olefine mit innenständigen Doppelbindungen und lineare Olefine reaktiver als verzweigte. Speziell im Falle der C₄-Olefine gilt, 1-Buten ist reaktiver als Isobuten und Isobuten ist reaktiver als die beiden 2-Butene (cis bzw. trans). Diese unterschiedliche Reaktivität kann genutzt werden, um einen hohen Anteil an Produkten, die durch endständige Hydroformylierung entstanden sind, zu gewinnen, d. h., aus 1-Buten soll hauptsächlich Valeraldehyd und nicht 2-Methylbutanal, aus Isobuten 3-Methylbutanal und nicht 2,2-Dimethylpropanal sowie aus den beiden 2-Butenen möglichst viel Valeraldehyd (n-Pentanal) und wenig 2-Methylbutanal entstehen.

Da es bislang keinen Katalysator gibt, der simultan mit zufriedenstellender Geschwindigkeit sowohl die Umsetzung von 1-Buten als auch von Isobuten und den 2-Butenen zu Produkten, die durch endständige Hydroformylierung entstanden sind, bewirkt, wird die Hydroformylierung, insbesondere wenn die Einsatzgemische sowohl Isobuten und/oder 1-Buten als auch 2-Butene enthalten, vorzugsweise zumindest zweistufig durchgeführt. Wird das erfindungsgemäße Verfahren zweistufig durchgeführt, wird vorzugsweise in einer Stufe Isobuten und/oder 1-Buten und in der anderen Stufe 2-Butene hydroformyliert.

In einer ersten Stufe wird die Hydroformylierung vorzugsweise mit einem geeigneten Katalysator unter Bedingungen vorgenommen, bei denen nur α-Olefine (1-Buten, Isobuten), jedoch nicht die 2-Butene zu den entsprechenden Aldehyden umgesetzt werden. Dabei werden die Bedingungen vorzugsweise derart gewählt, dass 1-Buten möglichst selektiv zu Valeraldehyd und Isobuten möglichst selektiv zu 3-Methylbutanal umgesetzt wird. Als Katalysatoren können beispielsweise Verbindungen eingesetzt werden, die Rhodium und triorganische Phosphorverbindungen, insbesondere Phosphine als Liganden aufweisen. Die Umsetzung kann in homogener Phase (analog dem UCC-Verfahren, beschrieben in EP 0 562 451) oder in heterogener Phase (analog dem Rhone-Poulenc-Ruhrchemie-Verfahren, beschrieben in DE 26 27 354 und EP 0 562 451) durchgeführt werden. Wegen der leichteren Katalysatorabtrennung wird die erste Stufe des Verfahrensschrittes a) bevorzugt gemäß dem zweiten Verfahren durchgeführt. Die Reaktionstemperaturen für die erste Stufe des Verfahrensschrittes a) betragen bevorzugt von 70 bis 150 °C, vorzugsweise von 100 bis 130 °C. Die Verfahrensdrücke betragen vorzugsweise von 2 bis 20 MPa, bevorzugt von 3 bis 6 MPa.

Optional kann die Hydroformylierung der 1-Olefine im Mehrphasensystem, wobei Edukt, Produkt und Synthesegas in einer kontinuierlichen Katalysatorphase dispergiert sind, unter hohen Leerrohrgeschwindigkeiten durchgeführt werden. Solche Verfahren werden beispielsweise in DE 199 25 384 A1 und DE 199 57 528 A1 beschrieben, auf welche hier ausdrücklich verwiesen wird.

Die Hydroformylierung der 1-Olefine in der ersten Stufe des Verfahrensschrittes a) kann einstufig oder zweistufig durchgeführt werden. Bei der zweistufigen Hydroformylierung wird im ersten Reaktor vorwiegend 1-Buten und im zweiten Reaktor hauptsächlich Isobuten umgesetzt. In beiden Reaktoren können die gleichen Katalysatoren oder unterschiedliche Katalysatoren eingesetzt werden. Bei Verwendung gleicher Katalysatoren ist eine gemeinsame Katalysatoraufarbeitung möglich.

Nach der gerade beschriebenen Hydroformylierung von 1-Buten und Teilen des Isobutens in der ersten Stufe des Verfahrensschrittes a) verbleiben im Einsatz-Kohlenwasserstoffgemisch falls vorhanden, die 2-Butene und gegebenenfalls Isobuten und höchstens Spuren von 1-Buten. Dieses Gemisch kann als solches unter Verwendung eines anderen Katalysatorsystems oder nach Auftrennung in zwei Fraktionen, von denen eine Isobuten und die andere die beiden 2-Butene enthält, hydroformyliert werden. Bevorzugt wird das Gemisch aufgetrennt und die Isobuten aufweisende Fraktion und die 2-Butene aufweisende Fraktion werden getrennt hydroformyliert.

Das Isobuten bzw. die Isobuten aufweisende Fraktion kann in hohen Selektivitäten zu 3-Methylbutanal hydroformyliert werden. Geeignete Katalysatoren dafür sind Rhodiumkomplexe, die ein- oder mehrzähnige Phosphitliganden enthalten. Geeignete einzähnige Phosphitliganden sind beispielsweise Triarylphosphite, deren Arylgruppen sowohl in ortho-Stellung zum Phosphit-Sauerstoff eine sperrige Gruppe aufweisen als auch in m- oder p-Stellung substituiert sind, wie z. B. Tris(2,4-di-tert.-butyl-phenyl)phosphit Die Hydroformylierung von Isobuten unter Verwendung eines Katalysatorsystems, das aus Rhodium und einem Bisphosphit besteht, wird beispielsweise in den Patentschriften US 4,668,651, US 4,769,498 und WO 85/03702 beschrieben, auf welche ausdrücklich verwiesen wird und deren Offenbarungsgehalt Gegenstand der vorliegenden Beschreibung ist.

Optional kann die abgetrennte Isobutenfraktion ganz oder teilweise in die vorgeschaltete erste Hydroformylierungsstufe zurückgeführt werden. Dabei kann es besonders vorteilhaft sein, vom Isobuten die gesättigten Kohlenwasserstoffe abzutrennen, was z. B. thermisch erfolgen kann. Nach einer solchen Abtrennung der gesättigten Kohlenwasserstoffe kann es besonders vorteilhaft sein, das Isobuten vollständig in die vorgeschaltete erste Hydroformylierungsstufe zurückzuführen.

Die Hydroformylierung von 2-Butenen bzw. 2-Butenen aufweisenden Fraktionen kann mit Hilfe von verschiedenen bekannten Katalysatoren durchgeführt werden, wobei üblicherweise ein Gemisch aus 2-Methylbutanal und Valeraldehyd entsteht. In den meisten Fällen ist 2-Methylbutanal das Hauptprodukt. Die Verwendung von unmodifizierten Kobaltkatalysatoren als Katalysator für die Hydroformylierung von 2-Butenen wird in EP 0 646 563 und die Verwendung von unmodifiziertem Rhodium wird in EP 0 562 451 beschrieben. Weiterhin kann für die Hydroformylierung von 2-Butenen das gleiche Katalysatorsystem, das für die Hydroformylierung von Isobuten eingesetzt wird, nämlich ein Komplex aus Rhodium und einzähniges Triarylphosphit verwendet werden. Hohe Selektivitäten an Valeraldehyd können bei der Verwendung eines Katalysators, bestehend aus Rhodium und sperrigen aromatischen Bisphosphiten, erhalten werden, wie sie beispielsweise in EP 0 213 639, EP 0 214 622 oder US 5,763,680 beschrieben werden. Allerdings sind die Reaktionsgeschwindigkeiten für ein technisches Verfahren relativ gering. Besonders bevorzugt wird als Bisphosphit-Ligand der in US 5,763,680 als Ligand D bezeichnete Ligand eingesetzt.

Wie oben ausgeführt ist, können die im Einsatzstoff vorliegenden Olefine getrennt oder gemeinsam hydroformyliert werden. Wenn der Linearität der Endprodukte keine große Bedeutung zukommt, ist es zweckmäßig, die Olefine gemeinsam zu hydroformylieren. Ist dagegen ein möglichst wenig verzweigtes Endprodukt erwünscht, ist es bevorzugt, die Hydroformylierung in mindestens zwei Stufen durchzuführen. Im Falle eines C₄-Olefingemisches bedeutet der letztere Fall, dass im ersten Reaktor 1-Buten und gegebenenfalls Isobuten umgesetzt wird und in dem/den nachgeschalteten Reaktor(en) optional die restlichen Olefine.

Aus den Hydroformylierungsgemischen kann der Katalysator nach bekannten Verfahren abgetrennt werden. Beispielsweise kann bei Verfahren, bei denen der Rhodium-Katalysator homogen im Reaktionsgemisch vorliegt, der Katalysator destillativ abgetrennt werden. Bei der Umsetzung in heterogener Phase (zwei flüssige Phasen) kann die Abtrennung des Katalysators z. B. durch Phasentrennung erfolgen (Ed. B. Cornils, W. A. Herrmann, Applied Homogeneous Catalysis with Organic Compounds, Vol. 1, S. 80, VCH-Verlag, 1996).

### Verfahrensschritt b)

Die Hydroformylierungsgemische können nach der Entkatalysierung entweder direkt in der Hydrierung eingesetzt werden oder aber zuvor destillativ oder mit anderen Trennmethoden in zwei oder mehrere Fraktionen aufgetrennt werden. Insbesondere kann es vorteilhaft sein, das Hydroformylierungsgemisch so aufzuarbeiten, dass eine oder mehrere im Wesentlichen Aldehyde aufweisende Fraktionen erhalten werden.

Die entkatalysierten Hydroformylierungsgemische oder die daraus durch ein Trennverfahren wie z. B. Destillation abgetrennten Aldehyde oder Aldehyd aufweisenden Fraktionen werden erfindungsgemäß hydriert. Dabei können die Hydroformylierungsgemische getrennt oder gemeinsam hydriert werden. Durch die Hydrierung entstehen aus den Aldehyden die entsprechenden gesättigten Alkohole. Dies sind beispielsweise Butanole, n-Pentanol, 2-Methylbutanol und 3-Methylbutanol.

Zur Hydrierung können z. B. Nickel-, Kupfer-, Kupfer/Nickel-, Kupfer/Chrom-, Kupfer/Chrom/Nickel-, Zink/Chrom-, Nickel/Molybdän-Katalysatoren verwenden werden. Die Katalysatoren können trägerfrei sein, oder die hydrieraktiven Stoffe bzw. ihre Vorläufer können auf Trägern, wie beispielsweise Siliziumdioxid oder Aluminiumdioxid, aufgebracht sein. Bevorzugte Katalysatoren, die in Verfahrensschritt b) eingesetzt werden und an denen die Hydroformylierungsgemische hydriert werden können, weisen jeweils 0,3 bis 15 Massen-% Kupfer und Nickel sowie als Aktivatoren 0,05 bis 3,5 Massen-% Chrom und optional 0,01 bis 1,6 Massen-%, vorzugsweise 0,02 bis 1,2 Massen-% einer Alkalikomponente auf einem Trägermaterial, vorzugsweise Aluminiumoxid und Siliziumdioxid auf. Die Mengenangaben beziehen sich auf den noch nicht reduzierten Katalysator. Die Alkalikomponente ist optional. Die Katalysatoren werden vorteilhaft in einer Form eingesetzt, in der sie einen geringen Strömungswiderstand bieten, z. B. in Form von Granalien, Pellets oder Formkörpern, wie Tabletten, Zylindern, Strangextrudaten oder Ringen. Sie werden zweckmäßig vor ihrem Einsatz aktiviert, z. B. durch Erhitzen im Wasserstoffstrom.

Die Hydrierung kann eine Gasphasen- oder Flüssigphasenhydrierung sein. Bevorzugt wird die Hydrierung bei einem Gesamtdruck von 0,5 bis 50 MPa, vorzugsweise von 1,5 bis 10 MPa durchgeführt. Eine Hydrierung in der Gasphase kann auch bei niedrigeren Drücken durchgeführt werden, wobei dann entsprechend große Gasvolumina vorliegen. Werden mehrere Hydrierungsreaktoren eingesetzt, können die Gesamtdrücke in den einzelnen Reaktoren innerhalb der genannten Druckgrenzen gleich oder verschieden sein. Die Reaktionstemperaturen können bei der Hydrierung in flüssiger oder gasförmiger Phase in der Regel von 120 bis 220 °C, insbesondere von 140 bis 180 °C betragen. Solche Hydrierungen sind beispielsweise in den Patentanmeldungen DE 198 42 369 und DE 198 42 370 beschrieben, auf welche hier ausdrücklich verwiesen wird.

Die Hydrierung wird bevorzugt in Gegenwart von Wasser durchgeführt. Das benötigte Wasser kann im Reaktorzulauf enthalten sein. Es ist jedoch auch möglich, Wasser an geeigneter Stelle in die Hydrierapparatur einzuspeisen. Bei Gasphasenhydrierung wird Wasser zweckmäßig in Form von Wasserdampf zugeführt. Ein bevorzugtes Hydrierverfahren ist die Flüssigphasenhydrierung unter Zusatz von Wasser, wie sie beispielsweise in DE 100 62 448 beschrieben ist. Besonders bevorzugt wird die Hydrierung bei einem Wassergehalt von 0,05 bis 10 Massen-%, insbesondere 0,5 bis 5 Massen-%, ganz besonders 1 bis 2,5 Massen-% durchgeführt. Der Wassergehalt wird dabei im Hydrieraustrag bestimmt.

Die aus der Hydrierung erhaltenen Gemische können entweder direkt zur Umsetzung mit Citronensäure oder Citronensäurederivat eingesetzt werden oder aber destillativ oder mit anderen Trennmethoden in zwei oder mehrere Fraktionen aufgetrennt werden. Insbesondere kann es vorteilhaft sein, das Hydrierungsgemisch so aufzuarbeiten, dass eine oder mehrere Fraktionen von Alkoholen mit der gleichen Anzahl an Kohlenstoffatomen erhalten werden. Bevorzugt kann die destillative Aufarbeitung so durchgeführt werden, dass es zu einer weitgehenden Auftrennung in die einzelnen Bestandteile kommt.

Wenn ein Citronensäureester von linearen Alkoholen als Einsatzalkoholen hergestellt werden soll, kann das lineare n-Pentanol von den verzweigten Pentanolen abgetrennt werden.

### Verfahrensschritt c) Umsetzung von Citronensäure oder Citronensäurederivat mit C₅-Alkohol zum Tripentylcitrat

Die Umsetzung der C₅-Alkohole zum entsprechenden Tripentylcitrat kann durch Reaktion mit Citronensäure, welche z. B. in der Form des Monohydrats oder wasserfrei (Anhydrat) eingesetzt werden kann, oder einem Derivat der Citronensäure, insbesondere einem Citronensäureester, erfolgen. Bevorzugt wird eine Veresterung von Citronensäure oder eine Umesterung von Citronensäureestern mit dem aus Schritt b) erhaltenen Alkohol durchgeführt.

Die erfindungsgemäßen Citronensäureester können z. B. durch Veresterung von Citronensäure, welche z. B. in der Form des Monohydrats oder wasserfrei (Anhydrat) eingesetzt werden kann, mit den entsprechenden Alkoholen erhalten werden. Der zur Bildung des Esters eingesetzte Alkohol bzw. das Alkoholgemisch, das gleichzeitig als Schleppmittel zur Abtrennung des bei der Reaktion entstehenden Wassers dienen kann, wird vorzugsweise im Überschuss, bevorzugt mit einem Überschuss von 5 bis 50 %, insbesondere 10 bis 40 %, besonders bevorzugt 15 bis 35 % der zur Bildung des Esters notwendigen molaren Menge eingesetzt.

Die Veresterung wird vorzugsweise in Gegenwart eines Veresterungskatalysators durchgeführt. Als Veresterungskatalysatoren können prinzipiell Säuren, wie beispielsweise Schwefelsäure, Sulfonsäuren, wie z. B. Methansulfonsäure oder p-Toluolsulfonsäure, Mineralsäuren oder Metalle oder deren Verbindungen eingesetzt werden. Geeignet sind z. B. Zinn, Titan, Zirkonium, die als feinverteilte Metalle oder zweckmäßig in Form ihrer Salze, Oxide oder in Form von löslichen organischen Verbindungen verwendet werden können. Allerdings sind die Metallkatalysatoren im Vergleich zu den Katalysatoren auf Basis von Protonensäuren Hochtemperaturkatalysatoren, die ihre volle Aktivität oft erst bei Temperaturen oberhalb 180 °C erreichen. Bevorzugt werden Schwefelsäure oder organische Sulfonsäuren eingesetzt, besonders bevorzugt Methansulfonsäure.

Die Katalysatorkonzentration kann abhängig von der Art des Katalysators in weiten Bereichen variiert werden. Für Protonensäuren sind Konzentrationen von 0,05 bis 2 Massen-%, bevorzugt 0,1 bis 1 Massen-%, besonders bevorzugt 0,15 bis 0,5 Massen-% vorteilhaft. Höhere Konzentrationen erhöhen zwar die Reaktionsgeschwindigkeit, können allerdings auch zur erhöhten Nebenproduktbildung, beispielsweise durch Wasserabspaltung beitragen.

Wie bereits in den oben genannten Publikationen erwähnt, neigen Citronensäure bzw. ihre Alkylester bei höherer Temperatur (> 153 °C) zur Wasserabspaltung unter Bildung von Aconitsäure bzw. deren Estern (Aconitate). Daher wurden die oben beschriebenen Verfahren in der Regel bei Temperaturen unterhalb von 150 °C betrieben. Daher wurden auch nur in wenigen Fällen Metall-basierte Säuren wie beispielsweise Tetrabutylorthotitanat für diese Aufgabe eingesetzt

Bei Temperaturen unterhalb von 150 °C kann beispielsweise mit den oben beschriebenen Protonensäuren gearbeitet werden, allerdings liegt dann die Reaktionszeit für die Veresterung von Citronensäure-Monohydrat mit beispielsweise n-Butanol häufig bei über 10 Stunden (WO 03/008369).

Es wurde nun überraschend gefunden, dass die Veresterung von Citronensäure, bevorzugt wasserfreier Citronensäure und besonders bevorzugt Citronensäure-Monohydrat mit den nach Verfahrensschritt b) hergestellten Pentanolen oder Pentanolgemischen bei Katalyse mit Protonensäuren, von denen Methansulfonsäure besonders bevorzugt ist, bereits nach 8 Stunden bei 155 bis 165 °C nahezu quantitativ verlaufen war und das erhaltene Produkt nach üblicher Aufarbeitung Reinheiten aufwies, die mit dem Stand der Technik, insbesondere mit den in WO 03/008369 angegebenen, vergleichbar waren.

Im Allgemeinen hängen die optimalen Temperaturen für die Durchführung der Veresterung von den Einsatzstoffen, von dem Reaktionsfortschritt und von der Katalysatorkonzentration und -art ab. Die optimalen Temperaturen können für jeden Einzelfall durch einfache Vorversuche leicht ermittelt werden. Durch die Verwendung von höheren Temperaturen kann die Reaktionsgeschwindigkeit erhöht werden, allerdings werden Nebenreaktionen begünstigt, wie beispielsweise Wasserabspaltung aus Alkoholen oder Bildung farbiger Nebenprodukte.

Im Falle der erfindungsgemäßen Pentylcitrate beträgt die Reaktionstemperatur vorzugsweise von 120 bis 180 °C, bevorzugt von 130 bis 170 °C und besonders bevorzugt von 155 bis 165 °C.

Die gewünschte Reaktionstemperatur bzw. der gewünschte Temperaturbereich kann durch Anpassung des Drucks im Reaktionsgefäß eingestellt werden. Vorzugsweise wird die Veresterung in Rahmen der vorliegenden Erfindung bei einem Druck von 0,1 MPa bis 1 hPa durchgeführt.

Zur Entfernung des Reaktionswassers kann es vorteilhaft sein, wenn das Wasser als Gemisch, z. B. als azeotropes Gemisch mit dem Alkohol aus dem Reaktionsgemisch abdestilliert wird. Die in die Reaktion zurückzuführende Flüssigkeitsmenge kann teilweise oder vollständig aus Alkohol bestehen, der durch Aufarbeitung des Destillats gewonnen wird. Es ist auch möglich, die Aufarbeitung zu einem späteren Zeitpunkt durchzuführen und die entfernte Flüssigkeitsmenge ganz oder teilweise durch frischen Alkohol, d. h. aus einem im Vorratsgefäß bereitstehenden Alkohol zu ersetzen. Bevorzugt wird soviel Flüssigkeitsmenge, wie sie aus dem Reaktionsgemisch entfernt wird durch Alkohol ersetzt.

Die Rohestergemische, die neben dem/den Ester(n), Alkohol, Katalysator oder dessen Folgeprodukten und gegebenenfalls Nebenprodukte enthalten, können nach an sich bekannten Verfahren aufgearbeitet werden. Die Aufarbeitung umfasst dabei vorzugsweise folgende Schritte: Abtrennung des überschüssigen Alkohols und gegebenenfalls Leichtsieder, Waschen des Rohprodukts mit Wasser oder einer wässrigen Salzlösung, Neutralisation der nicht umgesetzten Säuren, optional eine Wasserdampfdestillation, Umwandlung des Katalysators in einen leichtfiltrierbaren Rückstand, Abtrennung der Feststoffe und gegebenenfalls eine Trocknung. Dabei können je nach angewendetem Aufarbeitungsverfahren die Reihenfolge dieser Schritte verschieden sein.

Optional kann der gewünschte Ester aus dem Reaktionsgemisch, gegebenenfalls nach Neutralisation des Ansatzes, destillativ ab- bzw. aufgetrennt werden. Dies kann insbesondere im Fall von Produkten, die bei Raumtemperatur fest sind, vorteilhaft sein.

Die erfindungsgemäßen Citronensäuretripentylester können in einer anderen Ausführungsfom des erfindungsgemäßen Verfahrens durch Umesterung eines Citronensäureesters mit einem Einsatzalkohol, ausgewählt aus isomerenreinen Pentanolen oder einem geeigneten Pentanol-Isomerengemisch gewonnen werden. Als Edukte werden Citronensäureester eingesetzt, deren am O-Atom der Estergruppe gebundener Alkylrest vorzugsweise 1 bis 3 C-Atome aufweist. Dieser Rest kann aliphatisch, geradkettig oder verzweigt, alicyclisch oder aromatisch sein. Eine oder mehrere Methylengruppen dieses Alkyl-Rests können durch Sauerstoff substituiert sein. Es kann vorteilhaft sein, wenn die dem Eduktester zugrunde liegenden Alkohole niedriger sieden als die Einsatzalkohole. Ein bevorzugter Einsatzstoff ist Triethylcitrat, der industriell hergestellt wird und deshalb in großen Mengen zur Verfügung steht.

Die Umesterung wird vorzugsweise bei einer Temperatur von 100 bis 220°C durchgeführt. Die Temperatur wird besonders bevorzugt so hoch gewählt, dass der aus dem Eduktester entstehende Alkohole bei dem vorgegebenen Druck, vorzugsweise erhöhtem Druck, aus dem Reaktionsgemisch abdestilliert werden kann.

Die Aufarbeitung der Umesterungsgemische kann genauso wie für die Veresterungsgemische beschrieben erfolgen.

**Verfahrensschritt d): Carboxylierung der OH-Gruppe der Citronensäure bzw. des Citronensäureesters**

Die Umsetzung der OH-Gruppe der Citronensäure mit einer anderen Carbonsäure oder einem Anhydrid kann vor oder nach der Umsetzung, vorzugsweise nach der Umsetzung der Citronensäure bzw. des Citronensäurederivats mit dem Alkohol erfolgen. Die Umsetzung kann als einfache Veresterungsreaktion durchgeführt werden. Vorzugsweise erfolgt die Veresterung unter Einsatz von Alkansäuren wie Essigsäure, Propionsäure oder Buttersäure oder besonders bevorzugt unter Einsatz von Essigsäureanhydrid. Das Verfahren der Acetylierung des Citronensäureesters kann z. B. so wie in DE-AS 10 99 523 beschrieben erfolgen. Die Acetylierung umfasst vorzugsweise die Schritte Acetylierung mit einem Überschuß an Essigsäureanhydrid, Abdestillation von überschüssigem Essigsäureanhydrid sowie gegebenenfalls gebildeter Essigsäure, Neutralisation mit einer Base (z. B. Sodalösung, Natron- oder Kalilauge, Kalkmilch etc.) Waschung, Trocknung, Aufhellung (zum Beispiel durch Behandeln mit Bleicherde, Ozon oder Wasserstoffperoxid) und Filtration. Je nach Durchführung und Verlauf sind einige der hier genannten Schritte lediglich optional.

Die Carboxylierung, vorzugsweise die Acetylierung der OH-Gruppe der Citronensäure bzw. des Citronensäureesters, erfolgt bevorzugt im Anschluss an die Abtrennung des Alkoholüberschusses durch Destillation mit nachfolgender Wasserdampfbehandlung vom hergestellten Citronensäureester. Im Bedarfsfall können weitere Reinigungsschritte vor der Acetylierung durchgeführt werden, was aber bevorzugt nicht notwendig ist.

Im Falle der Acetylierung wird diese vorzugsweise durch Zugabe eines molaren Überschusses von Essigsäure oder bevorzugt Essigsäureanhydrid von 10 bis 80 %, bevorzugt 20 bis 50 % bei Temperaturen von 90 bis 120 °C, bevorzugt 100 bis 115 °C durchgeführt. Gleichzeitig mit oder nach Zugabe des Carboxylierungsmittels wird als Katalysator vorzugsweise eine Protonensäure zugegeben und die Reaktionsmischung bei dieser Temperatur eine gewisse Zeit, bevorzugt 30 Minuten bis zwei Stunden, insbesondere eine Stunde gerührt. Als Katalysator können hier verschiedenste Säuren eingesetzt werden. Bevorzugt werden Schwefelsäure oder Methansulfonsäure als Katalysator eingesetzt. Danach wird die überschüssige Säure oder das Anhydrid abgetrennt und es kann wie gewohnt aufgearbeitet (Neutralisation, gegebenenfalls Waschen, Wasserdampfdestillation, Trocknen, Filtration) werden.

Die erfindungsgemäßen Citronensäureester können als Weichmacher, insbesondere in Kunststoffzusammensetzungen, Klebstoffen, Dichtungsmassen, Lacken, Farben, Plastisolen, Kunstledern, Fußbodenbelägen, Unterbodenschutz, beschichteten Geweben, Tapeten oder Tinten als Weichmacher verwendet werden. Vorzugsweise können die erfindungsgemäßen Weichmacher in Profilen, Dichtungen, Lebensmittelverpackungen, Folien, Spielzeugen, Medizinalartikeln, Dachbahnen, Kunstledern, Fußbodenbelägen, Unterbodenschutz, beschichteten Geweben, Tapeten, Kabeln und Drahtummantelungen, besonders bevorzugt in Lebensmittelverpackungen, Spielzeugen, Medizinalartikeln, Tapeten und Fußbodenbelägen verwendet werden.

Unter Verwendung der erfindungsgemäßen Citronensäureester sind insbesondere erfindungsgemäße Zusammensetzungen erhältlich, die einen Citronensäureester enthalten.

Solche Zusammensetzungen können den erfindungsgemäßen Citronensäureester alleine oder in Mischungen mit anderen Weichmachern aufweisen. Falls die erfindungsgemäßen Zusammensetzungen die erfindungsgemäßen Citronensäureester im Gemisch mit anderen Weichmachern aufweisen, so können die anderen Weichmacher vorzugsweise aus der Gruppe der Phthalsäuredialkylester, bevorzugt mit 4 bis 13 C-Atomen in der Alkylkette; Trimellitsäuretrialkylester, bevorzugt mit 6 bis 10 C-Atomen in der Seitenkette; Adipinsäuredialkylester und bevorzugt Terephthalsäuredialkylester jeweils bevorzugt mit 4 bis 10 C-Atomen in der Seitenkette; 1,2-Cyclohexandisäurealkylestern, 1,3-Cyclohexandisäurealkylestern und 1,4-Cyclohexandisäurealkylestern, bevorzugt 1,2-Cyclohexandisäurealkylestern, jeweils bevorzugt mit Alkyl = Alkylrest mit 7 bis 10 Kohlenstoffatomen in der Seitenkette; Dibenzoesäurester von Glykolen; Alkylsulfonsäurester von Phenol mit vorzugsweise einem Alkylrest, der 8 bis 22 C-Atome enthält; Polymerweichmacher; Glycerinester und ganz besonders bevorzugt Citronensäuretrialkylester mit freier oder carboxylierter OH-Gruppe und einem Alkylrest von 4 oder 6 bis 10 C-Atomen und Benzoesäurealkylester, vorzugsweise mit 7 bis 13 C-Atomen in der Alkylkette, ausgewählt sein. In allen Fällen können die Alkylreste linear oder verzweigt und gleich oder verschieden sein. Besonders bevorzugt weist die Zusammensetzung neben Citronensäureestern insbesondere einen Benzoesäurealkylester mit Alkyl = Alkylrest mit 7 bis 13 Kohlenstoffatomen, vorzugsweise Benzoesäureisononylester, Benzoesäurenonylester, Benzoesäureisodecylester oder Benzoesäuredecylester auf. Der Anteil an erfindungsgemäßen Citronensäureestern in dem Gemisch mit anderen Weichmachern beträgt vorzugsweise 15 bis 90 % , besonders bevorzugt 20 bis 80 % und ganz besonders bevorzugt 30 bis 70 %, wobei sich die Massenanteile aller vorhandenen Weichmacher zu 100 % addieren.

Die genannten Zusammensetzungen aus Citronensäureester und anderen Weichmachern können als Weichmacherzusammensetzung in Kunststoffzusammensetzungen, Klebstoffen, Dichtungsmassen, Lacken, Farben, Plastisolen oder Tinten verwendet werden. Aus den erfindungsgemäßen Weichmacherzusammensetzungen hergestellte Kunststoffprodukte können beispielsweise sein: Profile, Dichtungen, Lebensmittelverpackungen, Folien, Spielzeug, Medizinalartikel, Dachbahnen, Kunstleder, Fußbodenbeläge, Unterbodenschutz, beschichtete Gewebe, Tapeten, Kabel und Drahtummantelungen. Bevorzugt sind aus dieser Gruppe Lebensmittelverpackungen, Spielzeug, Medizinalartikel, Tapeten und Fußbodenbeläge zu nennen.

Die erfindungsgemäßen Zusammensetzungen, die einen Citronensäureester enthalten, enthalten ein Polymer, ausgewählt aus Polyvinylchlorid (PVC), Polyvinylidenchlorid (PVDC), Polyacrylaten, insbesondere Polymethylmethacrylat (PMMA), Polyalkylmethacrylat (PAMA), Fluorpolymeren, insbesondere Polyvinylidenfluorid (PVDF), Polytetrafluorethylen (PTFE), Polyvinylacetat (PVAc, Polyvinylalcohol (PVA), Polyvinylacetale, insbesondere Polyvinylbutyral (PVB), Polystyrolpolymere, insbesondere Polystyrol (PS), Expandierbares Polystyrol (EPS), Acrylonitril-Styrol-Acrylat (ASA), Styrolacrylonitril (SAN), Acrylonitril-Butadien-Styrol (ABS), Styrol-Maleinsäureanhydrid-Copolymer (SMA), Styrol-Methacrylsäure-Copolymer, Polyolefine, insbesondere Polyethylen (PE) oder Polypropylen (PP), thermoplastische Polyolefine (TPO), Polyethylen-Vinylacetat (EVA), Polycarbonate, Polyethylenterephthalat (PET), Polybutylenterephthalat (PBT), Polyoxymethylen (POM), Polyamid (PA), Polyethylenglykol (PEG), Polyurethan (PU), Thermoplastisches Polyurethan (TPU), Polysulfide (PSu), Biopolymere, insbesondere Polymilchsäure (PLA), Polyhydroxylbuttersäure (PHB), Polyhydroxylvaleriansäure (PHV), Polyester, Stärke, Cellulose und Cellulose-Derivate, insbesondere Nitrocellulose (NC), Ethylcellulose (EC), Celluloseacetat (CA), Cellulose-Acetat/Butyrat (CAB), Gummi oder Silikone sowie Mischungen oder Copolymere der genannten Polymere oder deren monomeren Einheiten Vorzugsweise weisen die erfindungsgemäßen Zusammensetzungen PVC oder Homo- oder Copolymere auf Basis von Ethylen, Propylen, Butadien, Vinylacetat, Glycidylacrylat, Glycidylmethacrylat, Methacrylaten, Acrylaten, Acrylaten oder Methacrylaten mit am Sauerstoffatom der Estergruppe gebundenen Alkylresten von verzweigten oder unverzweigten Alkoholen mit einem bis zehn Kohlenstoffatome(n), Styrol, Acrylnitril oder cyclischen Olefinen auf.

Bevorzugt enthält die erfindungsgemäße Zusammensetzung als PVC-Typ Suspensions-, Masse-, Mikrosuspensions- oder Emulsions-PVC. Bezogen auf 100 Massenteile Polymer enthalten die erfindungsgemäßen Zusammensetzungen vorzugsweise von 5 bis 200, bevorzugt von 10 bis 150 Massenteile an Weichmacher.

Die erfindungsgemäßen Zusammensetzungen können neben den genannten Bestandteilen weitere Bestandteile enthalten, insbesondere z. B. weitere Weichmacher, Füllstoffe, Pigmente, Stabilisatoren, Co-Stabilisatoren wie beispielsweise epoxidiertes Sojabohnenöl, Gleitmittel, Treibmittel, Kicker, Antioxidanzien oder Biozide.

Die genannten Polymere aufweisenden Zusammensetzungen können als Kunststoffzusammensetzungen, Klebstoffe, Dichtungsmassen, Lacke, Farben, Plastisole, Kunstleder, Fußbodenbeläge, Unterbodenschutz, Gewebebeschichtungen, Tapeten oder Tinten oder zu deren Herstellung verwendet werden. Die genannten Zusammensetzungen können insbesondere Profile, Dichtungen, Lebensmittelverpackungen, Folien, Spielzeug, Medizinalartikel, Dachbahnen, Kunstleder, Fußbodenbeläge, Unterbodenschutz, beschichtete Gewebe, Tapeten, Kabel und Drahtummantelungen sein. Bevorzugt sind die Zusammensetzungen Lebensmittelverpackungen, Spielzeug, Medizinalartikel, Tapeten und Fußbodenbeläge zu nennen.

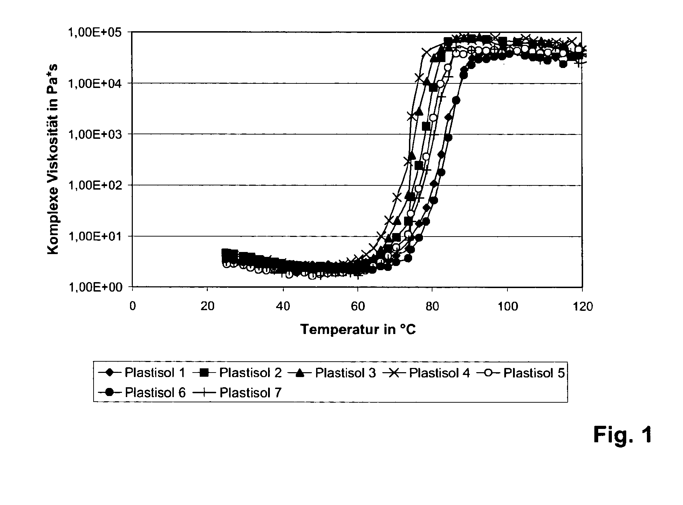

An Hand der Figur Fig. 1 wird die Erfindung näher erläutert, ohne dass die Erfindung auf die dort beispielhaft dargestellten Ausführungsarten beschränkt sein soll.

In Fig. 1 sind Gelierkurven für die in Beispiel 8 getesteten sieben Plastisole abgebildet.

Aufgetragen ist die komplexe Viskosität in Abhängigkeit von der Temperatur. Die Messwerte zu Plastisol 1 sind als Rauten gekennzeichnet, die Messwerte zu Plastisol 2 sind als Quadrate gekennzeichnet, die Messwerte zu Plastisol 3 sind als Dreiecke gekennzeichnet, die Messwerte zu Plastisol 4 sind als X gekennzeichnet, die Messwerte zu Plastisol 5 sind als offene Kreise gekennzeichnet, die Messwerte zu Plastisol 6 sind als ausgefüllte Kreise gekennzeichnet und die Messwerte zu Plastisol 7 sind als Kreuze gekennzeichnet.

Die folgenden Beispiele sollen die Erfindung erläutern, ohne die Anwendungsbreite einzuschränken, die sich aus der Beschreibung und den Patentansprüchen ergibt.

### Beispiele

Die in den Beispielen 1 und 2 beschriebenen Synthesen der Citronensäureester wurden ausgehend von über den Handel (FLUKA) erhältlichen Pentanolen, nämlich n-Pentanol (GC-Reinheit > 99 Massen-%), 2-Methylbutanol (> 98 Massen-%) und 3-Methylbutanol (> 98,5 Massen-%) hergestellt. Für die Herstellung der verschiedenen Ester wurden die in der Tabelle 1 angegebenen Alkohole bzw. Alkoholgemische eingesetzt.

**Tabelle 1: In den Beispielen 1 und 2 eingesetzte Alkohole bzw. Alkoholgemische. Die %-Angaben sind Massen-%.**

| **n-Pentanol** | **2-Methylbutanol** | **3-Methylbutanol** | **Entsprechender Ester** | **Nummer** |
|---|---|---|---|---|
| 100 % | 0 % | 0 % | Tri-n-pentylcitrat | 1 A |
| 100 % | 0 % | 0 % | Acetyl-tri-n-pentylcitrat | 2 A |
| 0 % | 0 % | 100 % | Acetyl-tri-(3-methylbutyl)citrat | 2 B |
| 90 % | 10 % | 0 % | Acetyl-tripentylcitrat | 2 C |

Da es sich hierbei um isomere Verbindungen handelt, die keiner Anpassung der stöchiometrisch notwendigen Mengen bedürfen, wird im Folgenden vereinfachend nur allgemein von Pentanolen gesprochen.

### Beispiel 1: Herstellung von Tripentylcitraten (Vergleich)

In einem 2 Liter-Mehrhalskolben mit Rührer, Wasserabscheider, Tropftrichter, Innenthermometer und Tauchrohr wurden 210 g (1 Mol) Citronensäuremonohydrat (Fa. Riedel de Haen, Reinheit > 99,5 Massen-%) und zunächst 300 g von insgesamt 352 g Pentanol oder Pentanolgemisch (4 Mol) gemäß Tabelle 1 vorgelegt. Unter Rühren wurde zunächst 30 Minuten mit Stickstoffgas über das Tauchrohr gespült und dann langsam aufgeheizt. Ab ca. 115 °C fiel zunächst das Kristallwasser aus der Säure an, welches über den Wasserabscheider abgenommen wird. Bei Erreichen einer Temperatur von 145 °C wurden über den Tropftrichter (Stickstoffatmosphäre) 0,63 g Methansulfonsäure gelöst in den noch fehlenden 52 g Pentanol bzw. Pentanolgemisch zugegeben. Nach Erreichen der Reaktionstemperatur von 160 °C wurde durch sukzessive Druckverringerung ein konstanter Rücklauf des Pentanol/Wasser-Gemisches eingestellt. Nach ca. 8 Stunden betrug die Säurezahl < 1 mg KOH/g (DIN EN ISO 2114) und die Veresterung war abgeschlossen.

Anschließend wurde - weiterhin unter Stickstoff-Atmosphäre - der Wasserabscheider durch eine Destillationsbrücke ersetzt und bei langsam gesteigertem Vakuum bis 160 °C der Alkoholüberschuss abdestilliert.

Zur Aufarbeitung wurde der Ansatz auf 100 °C abgekühlt. Danach wurden zum Reaktionsgemisch 200 ml 5-Massen-%ige Kochsalzlösung gegeben und die Mischung 15 Minuten bei 80 °C gerührt. Danach wurde die wässrige Phase abgetrennt und der Ansatz noch ein weiteres Mal mit der gleichen Menge Kochsalzlösung gewaschen und nochmals die Phasen getrennt. Nach dem zweiten Waschvorgang wurde gemäß DIN EN ISO 2114 die Säurezahl bestimmt und bei 80 °C mit der neunfachen stöchiometrischen Menge an 5 Massen-%iger Natronlauge 30 Minuten unter Rühren neutralisiert. Danach wurde die wässrige Phase abgelassen und nochmals wie oben beschrieben zwei Mal mit 5 Massen-%iger Kochsalzlösung gewaschen.

Nach der Phasentrennung wurde das Rohestergemisch wieder auf 160 °C erhitzt und bei dieser Temperatur unter Vakuum langsam 8 Massen-% vollentsalztes Wasser, bezogen auf die erwartete Rohestermenge über das Tauchrohr zugetropft. Hierbei wurde darauf geachtet, dass die Temperatur nicht über 160 °C anstieg. Anschließend wurde mit Stickstoff belüftet, 2 Massen-% gepulverte Aktivkohle zugegeben und unter erneutem Vakuum (bis 5 hPa) auf 80 °C abgekühlt und das Produkt anschließend filtriert.
Reinheit: 99 Flächen-% (ermittelt durch Gaschromatographie)

### Beispiel 2: Herstellung von Acetyltripentylcitraten

In einem 2 Liter-Mehrhalskolben mit Rührer, Wasserabscheider, Tropftrichter, Innenthermometer und Tauchrohr wurden 210 g (1 Mol) Citronensäure-monohydrat (Fa. Riedel de Haen) und zunächst 300 von insgesamt 352 g Pentanol oder Pentanolgemisch (4 Mol) gemäß Tabelle 1 vorgelegt. Unter Rühren wurde zunächst 30 Minuten mit Stickstoffgas über das Tauchrohr gespült und dann langsam aufgeheizt. Ab ca. 115 °C fiel zunächst das Kristallwasser aus der Säure an, welches über den Wasserabscheider abgenommen wird. Bei Erreichen einer Temperatur von 145 °C wurden über den Tropftrichter (Stickstoffatmosphäre) 0,63 g Methansulfonsäure gelöst in den noch fehlenden 52 g Pentanol bzw. Pentanolgemisch zugegeben. Nach Erreichen der Reaktionstemperatur von 160 °C wurde durch sukzessive Druckverringerung ein konstanter Rücklauf des Pentanol/Wasser-Gemisches eingestellt. Nach ca. 8 Stunden war die Säurezahl < 1 mg KOH/g (DIN EN ISO 2114) und die Veresterung abgeschlossen.

Anschließend wurde - weiterhin unter Stickstoff-Atmosphäre - der Wasserabscheider durch eine Destillationsbrücke ersetzt und bei langsam gesteigertem Vakuum bis 160 °C der Alkoholüberschuss abdestilliert.

Anschließend wurde unter Stickstoffatmosphäre der Wasserabscheider durch eine Destillationsbrücke ersetzt und die Apparatur wieder unter Vakuum genommen. Über den Tropftrichter und das Tauchrohr wurden dann 8 Massen-% vollentsalztes Wasser, bezogen auf die erwartete Rohestermenge zugetropft. Nach vollständiger Zugabe des Wassers wurde unter Vakuum (< 5 mbar) auf 110 °C abgekühlt.

Zur Acetylierung wurde die 1,25-fache molare Menge an Essigsäureanhydrid verwendet. Zur Berechnung wurde die theoretische Menge an Rohester herangezogen.

Die Apparatur wurde zur Zugabe des Essigsäureanhydrids mit Stickstoff belüftet und 5 Minuten mit Stickstoff gespült. Das Essigsäureanhydrid wurde über den Tropftrichter langsam zugegeben. Dann wurden bei 100 °C bis 110 °C langsam 0,5 g Methansulfonsäure zugegeben und es wurde noch eine Stunde weitergerührt. Danach wurde unter vorsichtigem Vakuum bei maximal 130 °C Essigsäure und überschüssiges Essigsäureanhydrid abdestilliert.

Zur Aufarbeitung wurde der Ansatz auf 100 °C abgekühlt. Danach wurden zum Reaktionsgemisch 200 ml 5 Massen-%ige Kochsalzlösung gegeben und die Mischung 15 Minuten bei 80 °C gerührt. Danach wurde die wässrige Phase abgetrennt und der Ansatz noch ein weiteres Mal mit der gleichen Menge Kochsalzlösung gewaschen und nochmals die Phasen getrennt. Nach dem zweiten Waschvorgang wurde gemäß DIN EN ISO 2114 die Säurezahl bestimmt und bei 80 °C mit der neunfachen stöchiometrischen Menge an 5 Massen-%iger Natronlauge 30 Minuten unter Rühren neutralisiert. Danach wurde die wässrige Phase abgelassen und nochmals wie oben beschrieben zwei Mal mit 5 Massen-%iger Kochsalzlösung gewaschen.

Nach der Phasentrennung wurde das Rohestergemisch wieder auf 160 °C erhitzt und bei dieser Temperatur unter Vakuum langsam 8 Massen-% vollentsalztes Wasser, bezogen auf die erwartete Rohestermenge über das Tauchrohr zugetropft. Hierbei wurde darauf geachtet, dass die Temperatur nicht über 160 °C anstieg. Anschließend wurde mit Stickstoff belüftet, 2 Massen-% gepulverte Aktivkohle zugegeben und unter erneutem Vakuum (bis 5 hPa) auf 80 °C abgekühlt, bei dieser Temperatur etwa 30 Minuten mit 2 Massen-% Wasserstoffperoxid gerührt, anschließend bei 120 °C getrocknet und dann wiederum abgekühlt und filtriert.

### Beispiel 3: Herstellung der Plastisole

Die verwendeten Einwaagen der Komponenten für die verschiedenen Plastisole sind der nachfolgenden Tabelle 2 zu entnehmen.

**Tabelle 2: Rezepturen gemäß Beispiel 3 (Alle Angaben in phr (= Massenteile pro 100 Massenteile PVC))**

| **Plastisolrezeptur** | **1** | **2** | **3** | **4** | **5** | **6** | **7** |
|---|---|---|---|---|---|---|---|
| Vestolit B 7021 (Vestolit GmbH) | 100 | 100 | 100 | 100 | 100 | 100 | 100 |
| Vestino19 (DINP der OXENO Olefinchemie) | 50 | | | | | | |
| DEHP (OXENO Olefinchemie GmbH) | | 50 | | | | | |
| Acetyl-tri-n-butylcitrat (Fa. Jungbunzlauer) | | | 50 | | | | |
| Tri-n-pentylcitrat (1 A) | | | | 50 | | | |
| Acetyl-tri-n-pentylcitrat (erfindungsgemäß, 2 A) | | | | | 50 | | |
| Acetyl-tri-(3-methylbutyl)-citrat (erfindungsgemäß, 2 B) | | | | | | 50 | |
| Acetyl-tripentylcitrat (erfindungsgemäß, 2 C) | | | | | | | 50 |
| Epoxidiertes Sojabohnenöl (Drapex 39, Fa. Crompton) | 3 | 3 | 3 | 3 | 3 | 3 | 3 |
| Mark CZ 140 (Fa. Crompton) | 1,5 | 1,5 | 1,5 | 1,5 | 1,5 | 1,5 | 1,5 |

Die Weichmacher wurden vor der Zugabe auf 25 °C temperiert. Zuerst wurden die flüssigen Bestandteile und dann die pulverförmigen in einen PE-Becher eingewogen. Von Hand wurde die Mischung mit einem Salbenspatel so eingerührt, dass kein unbenetztes Pulver mehr vorhanden war. Der Mischbecher wurde dann in die Klemmvorrichtung eines Dissolverrührers eingespannt. Vor dem Eintauchen des Rührers in die Mischung wurde die Drehzahl auf 1800 Umdrehungen pro Minute eingestellt. Nach dem Einschalten des Rührers wurde so lange gerührt, bis die Temperatur an der Digitalanzeige des Thermofühlers 30,0 °C erreichte. Damit war sicher gestellt, dass die Homogenisierung des Plastisols bei einem definierten Energieeintrag erreicht wurde. Danach wurde das Plastisol sofort bei 25,0 °C temperiert.

### Beispiel 4: Viskositätsmessungen und Lagerstabilitäten

Die Messung der Viskositäten der in Beispiel 3 hergestellten Plastisole wurden in Anlehnung an die DIN 53 019 mit einem Rheometer Physica DSR 4000 (Fa. Paar-Physica), welches über die zugehörige Software US 200 gesteuert wird, wie folgt durchgeführt.

Das Plastisol wurde im Vorratsbehälter nochmals mit einem Spatel umgerührt und in dem Messsystem Z3 (DIN 25 mm) gemäß Bedienungsanleitung vermessen. Die Messung verlief bei 25 °C automatisch über die o. g. Software. Folgende Punkte wurden angesteuert:
- Eine Vorscherung von 100 s⁻¹ für den Zeitraum von 60 s, bei der keine Messwerte aufgenommen wurden
- Eine Abwärtsrampe, beginnend bei 200 s⁻¹ bis herunter zu 0,1 s⁻¹, aufgeteilt in eine logarithmische Reihe mit 30 Schritten mit jeweils 5 s Messpunktdauer.

Die Aufbereitung der Messdaten wurde nach der Messung automatisch von der Software durchgeführt. Dargestellt wurde die Viskosität in Abhängigkeit von der Schergeschwindigkeit. Die Messungen wurden jeweils nach 2 h, 4 h, 24 h und 28 Tagen durchgeführt. Zwischen diesen Zeitpunkten wurde die Paste bei 25 °C gelagert.

In der nachfolgenden Tabelle 3 sind exemplarisch für die Schergeschwindigkeit von 100 s⁻¹ jeweils die nach den angegebenen Lagerzeiten erhaltenen entsprechenden Viskositätswerte aufgeführt.

**Tabelle 3: Schergeschwindigkeit 100 s⁻¹ (Angaben der Viskositäten in Pa*s)**

| **Plastisol rezeptur** | **Verwendeter Weichmacher** | **2 h** | **4 h** | **24 h** | **28 d** | **Gesamtanstieg von 2 h nach 28 Tagen in %** |
|---|---|---|---|---|---|---|
| 1 | DINP (Vestino19) | 3,59 | 3,68 | 6,51 | 8,22 | 129 |
| 2 | DEHP | 4,04 | 4,13 | 7,44 | 11,2 | 177 |
| 3 | Acetyl-tri-n-butylcitrat (Fa. Jungbunzlauer) | 2,84 | 2,87 | 5,67 | 8,58 | 202 |
| 4 | Tri-n-pentylcitrat (1 A) | 2,49 | 2,69 | 5,24 | 11,3 | 354 |
| 5 | Acetyl-tri-n-pentylcitrat (erfindungsgemäß, 2 A) | 2,63 | 2,70 | 4,80 | 6,63 | 152 |
| 6 | Acetyl-tri-3-methylbutylcitrat (erfindungsgem., 2 B) | 4,47 | 4,47 | 7,96 | 9,72 | 117 |
| 7 | Acetyl-tri-pentylcitrat (erfindungsgemäß, 2 C) | 2,81 | 2,83 | 4,93 | 6,81 | 142 |

### Beispiel 5: Herstellung von Gießlingen für Shore-Härte-Prüfungen

Die Shore-Härte A ist ein Maß für die Weichheit eines Probekörpers. Je weiter bei einer bestimmten Messdauer eine genormte Nadel in den Probenkörper eindringen kann, desto niedriger fällt der Messwert aus. Der Weichmacher mit der höchsten Effizienz ergibt bei gleicher Weichmachermenge den niedrigsten Wert für die Shore Härte. Umgekehrt kann bei sehr effizienten Weichmachern ein gewisser Anteil in der Rezeptur eingespart werden, was in vielen Fällen für den Verarbeiter geringere Kosten bedeutet.

Zur Bestimmung der Shore-Härten wurden die gemäß Beispiel 3 hergestellten Plastisole in kreisrunde Gießformen mit einem Durchmesser von 50 mm gegossen. Dann wurden die Plastisole in den Formen im Umlufttrockenschrank 10 min bei 200 °C geliert, nach Abkühlung entnommen und vor der Messung mindestens 16 Stunden im Normklima (23 °C; 50 % relative Feuchte) gelagert. Die Dicke der Scheiben betrug ca. 8 mm.

Die Messungen selbst wurden in Anlehnung an DIN 53 505 mit einem Shore-A-Messgerät der Fa. Zwick-Roell durchgeführt, der Messwert jeweils nach 3 Sekunden abgelesen. An jedem Probekörper wurden drei verschiedene Messungen an verschiedenen Stellen (nicht im Randbereich) durchgeführt und jeweils der Mittelwert notiert. Die erhaltenen Messwerte sind in Tabelle 4 aufgeführt.

**Tabelle 4: Ergebnisse der Shore-Härte A Bestimmung**

| **Plastisolrezeptur** | **Verwendeter Weichmacher** | **Shore-Härte A** |
|---|---|---|
| 1 | Vestinol 9 | 83 |
| 2 | DEHP | 81 |
| 3 | Acetyl-tri-n-butylcitrat (Fa. Jungbunzlauer) | 79 |
| 4 | Tri-n-pentylcitrat (1 A) | 76 |
| 5 | Acetyl-tri-n-pentylcitrat (erfindungsgemäß, 2 A) | 79 |
| 6 | Acetyl-tri-(3-methylbutyl)-citrat (erfindungsgemäß, 2 B) | 83 |
| 7 | Acetyltripentylcitrat (erfindungsgemäß, 2 C) | 79 |

### Beispiel 6: Bestimmung der Thermostabilität

Bei diesem Test wurden die Prüfkörper erhöhten Temperaturen ausgesetzt, die im Bereich der Verarbeitungstemperaturen liegen. Die Zeit bis zum Auftreten einer deutlichen Verfärbung dient als Maß für die thermische Stabilität der Rezeptur. Durch die Abspaltung von Chlorwasserstoff (HCl) und die Bildung von Polyensegmenten verfärben sich farblose Proben über Gelb und Braun nach Schwarz.

### Herstellung der Folien:

Zur Erzeugung der Prüfkörper wurden zunächst für jede Rezeptur 1 mm dicke Folien hergestellt. Hierzu wurde zunächst Hochglanztrennpapier (Fa. Sappi, Italien) auf eine Größe von 30 * 44 cm zugeschnitten und im Spannrahmen der Streicheinrichtung LTSV für den Mathis-Ofen eingelegt. Danach wurde der Spannrahmen auf den Führungsrahmen aufgelegt, der Mathis-Ofen (Typ LTF) auf 200 °C eingestellt und der Rahmen nach Erreichen dieser Temperatur 15 Sekunden vorgewärmt. Danach wurde die Rakel in die Einspannvorrichtung gelegt und der Rakelspalt über Vorversuche so eingestellt, dass die Foliendicke nach Abschluss der Gelierung 1 mm (+/- 0,05 mm) betrug. Auf den vorderen Rand des Papiers wurde ein Klebestreifen angebracht, um überschüssige Paste aufzufangen. Danach wurde die Paste vor der Rakel aufgetragen und diese durch Ziehen des Führungsrahmens mit der Rakel über das eingespannte Trennpapier verstrichen (ca. 6 m/min Geschwindigkeit). Danach wurde die Rakel herausgenommen und der Klebestreifen mit der überschüssigen Paste abgenommen. Anschließend wurde die Schmelzwalze abgesenkt und der Spannrahmen in den Ofen eingefahren. Nach der Gelierung (2 Minuten bei 200 °C) wurde der Rahmen wieder aus dem Ofen herausgefahren und nach Abkühlen die Folie von dem Papier abgezogen.

### Probenvorbereitung:

Die 1 mm dicken Folien wurden mit einer Schere auf ein Maß von 20 * 20 mm zugeschnitten. Pro Rezeptur wurden 15 Probekörper benötigt. Diese wurden dann hintereinander auf den Testrahmen aufgelegt. Die Temperatur des Mathis-Thermotesters wurde auf 200 °C eingestellt, der Testrahmen eingefahren und mit einer konstanten Geschwindigkeit wieder aus dem Ofen ausgefahren. Auf diese Art konnten im Abstand von 1,5 Minuten jeweils die Probekörper ausgefahren werden, so dass die thermische Belastung der Prüfkörper zeitlich definiert variiert werden konnte. Nach 23 Minuten waren (incl. Nullprobe) alle Prüfkörper wieder aus dem Ofen ausgefahren.

Die Prüfkörper wurden, geordnet nach Rezeptur und Verweilzeit im Ofen auf einen Karton zwecks Vergleichbarkeit geheftet.

Die Ergebnisse der Prüfung zur Thermostabilität sind in der nachfolgenden Tabelle 5 aufgeführt. Angegeben ist jeweils der Zeitpunkt bis zur intensiven Schwarzfärbung.

**Tabelle 5: Ergebnisse zur Bestimmung der Thermostabilität**

| **Plastisolrezeptur** | **Verwendeter Weichmacher** | **Zeitpunkt bis Schwarzfärbung in Minuten (Verweilzeit im Ofen)** |
|---|---|---|
| 1 | Vestinol 9 | 16,5 |
| 2 | DEHP | 16,5 |
| 3 | Acetyl-tri-nbutylcitrat (Fa. Jungbunzlauer) | 16,5 |
| 4 | Tri-n-pentylcitrat (1 A) | 12 |
| 5 | Acetyl-tri-n-pentylcitrat (erfindungsgemäß, 2 A) | 16,5 |
| 6 | Acetyl-tri-3-methylbutyl-citrat (erfindungsgemäß, 2 B) | 16,5 |
| 7 | Acetyl-tripentylcitrat (erfindungsgemäß, 2 C) | 15 |

Wie an Hand der Daten in Tabelle 5 leicht abgelesen werden kann, zeigt die Plastisolrezeptur, in der als Weichmacher ein Pentylcitrat verwendet wurde, welches eine freie OH-Gruppe aufweist, die schlechteste Thermostabilität.

### Beispiel 7: Messung der Flüchtigkeit aus der Folie in Anlehnung an DIN 53 407

Aus den wie in Beispiel 6 hergestellten Folien mit einer Dicke von etwa 1 mm werden jeweils drei Rundscheiben mit einem Durchmesser von 50 mm ausgestanzt und zunächst 24 h im Normklima (23 °C / 50 % relative Luftfeuchtigkeit) gelagert und dann verwogen. Danach werden die Rundscheiben in Anlehnung an die DIN 53 407 (Verfahren A, direkter Kontakt mit Aktivkohle, Körnung 2,5 mm) jeweils 24 Stunden im Heizschrank bei 80 °C erwärmt. Danach werden die Scheiben wieder dem Heizschrank entnommen, im Normklima bei 24 Stunden abgekühlt und wieder verwogen, bevor sie erneut im Heizschrank gelagert werden. Nach einer Heizperiode von 7 * 24 Stunden ist die Messung beendet. In Tabelle 6 sind die erhaltenen Messwerte aufgeführt:

**Tabelle 6: Ergebnisse der Flüchtigkeitsmessung**

| **Plastisolrezeptur** | **Verwendeter Weichmacher** | **1 d** | **2 d** | **3 d** | **4 d** | **5 d** | **6 d** | **7 d** |
|---|---|---|---|---|---|---|---|---|
| 1 | Vestinol 9 | 0,65 | 0,9 | 1,26 | 1,69 | 2,09 | 2,24 | 2,48 |
| 2 | DEHP | 1,28 | 2,04 | 2,89 | 3,88 | 4,80 | 5,41 | 6,11 |
| 3 | Acetyl-tri-n-butylcitrat (Fa. Jungbunzlauer) | 3,17 | 5,29 | 7,40 | 9,44 | 11,20 | 12,40 | 13,60 |
| 4 | Tri-n-pentylcitrat (1 A) | 1,43 | 2,12 | 3,02 | 4,09 | 5,18 | 5,70 | 6,35 |
| 5 | Acetyl-tri-n-pentylcitrat (erfindungsgemäß, 2 A) | 1,26 | 1,82 | 2,58 | 3,54 | 5,31 | 5,69 | 6,21 |
| 6 | Acetyl-tri-3-methylbutyl-citrat (erfindungsgemäß, 2 B) | 1,75 | 2,71 | 4,25 | 5,50 | 7,10 | 7,74 | 8,54 |
| 7 | Acetyl-tripentylcitrat (erfindungsgemäß, 2 C) | 1,15 | 1,70 | 2,53 | 3,51 | 4,43 | 4,99 | 5,56 |

Die Flüchtigkeit der Proben, die die erfindungsgemäßen Weichmacher Tri-n-pentylcitrat (1A) und Acetyltripentylcitrat (2A, 2C) enthalten, ist vergleichbar mit der des DEHP und bietet somit im Vergleich zum ATBC einen deutlichen Vorteil.

### Beispiel 8: Bestimmung des Gelierverhaltens

Die Untersuchung des Gelierverhaltens der Plastisole wurde in einem Oszillationsviskosimeter der Marke Bohlin CVO (Meßsystem PP20), welches schubspannungsgesteuert betrieben wurde, vorgenommen.

Folgende Parameter wurden eingestellt:

| | |
|---|---|
| Modus: | Temperatur-Gradient |
| | Start-Temperatur: 25 °C |
| | End-Temperatur: 180 °C |
| | Heiz/Kühlrate: 2 °C/min |
| | Temperatur nach der Messung: 25 °C |
| | Oszillations-Frequenz: 2 Hz |
| | Verzögerungszeit: 1 s |
| | Wartezeit: 15 s |
| | Kontinuierliche Oszillation: an |
| | Automatische Schubspannungsvorgabe: an |
| | Startschubspannung: 0,3 Pa |
| | Soll-Deformation: 0,002 |
| | Spaltweite 0,5 mm |

### Durchführung der Messung:

Auf die untere Meßsystemplatte wurde mit dem Spatel ein Tropfen der zu messenden Plastisolrezeptur luftblasenfrei aufgetragen. Dabei wurde darauf geachtet, dass nach dem Zusammenfahren des Messsystems etwas Plastisol gleichmäßig aus dem Messsystem herausquellen konnte (nicht mehr als ca. 6 mm rundum). Anschließend wurde die Schutzabdeckung, die auch der Wärmeisolierung dient, aufgelegt und die Messung gestartet.

Aufgetragen wurde die sog. komplexe Viskosität des Plastisols in Abhängigkeit von der Temperatur. Ein Einsetzen des Geliervorganges war in einem plötzlichen starken Anstieg der komplexen Viskosität zu erkennen. Je früher dieser Viskositätsanstieg einsetzte, desto besser war die Gelierfähigkeit des Systems.

In Figur Fig. 1 sind die Gelierkurven für die sieben Plastisole aufgeführt. Man kann erkennen, dass das Tripentylcitrat die beste Gelierleistung erbringt. Durch Acetylierung wird sie zwar etwas reduziert, ist aber nach wie vor vergleichbar mit der des DEHP und besser als die des DINP. Der anteilige Ersatz von 10 Mol-% n-Pentanol durch 2-Methylbutanol hat keinen signifikanten Einfluss. Acetyl-tri-(3-methylbutyl)citrat geliert praktisch gleich schnell wie DINP.

## Patentansprüche

1. Citronensäureester der Formel I, 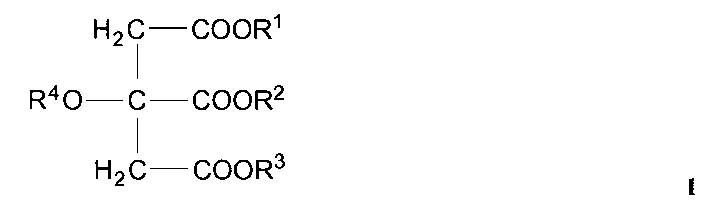 **dadurch gekennzeichnet,**
**dass** die Reste R¹, R² und R³ jeweils ein Alkylrest mit einer Anzahl an Kohlenstoffatomen von 5 sind und der Rest R⁴ ein Ameisensäurerest, ein Essigsäurerest, ein Propionsäurerest, ein Buttersäurerest oder ein Valeriansäurerest ist.

2. Citronensäureester nach Anspruch 1,
**dadurch gekennzeichnet,**
**dass** die Alkylreste R¹, R² und R³ eine längste Kohlenstoffkette von zumindest 4 Kohlenstoffatomen aufweisen und eine Gesamtzahl an Kohlenstoffatomen pro Alkylrest von 5 aufweisen.

3. Citronensäureester nach Anspruch 1 oder 2,
**dadurch gekennzeichnet,**
**dass** die Alkylreste R¹, R² und R³ zu mehr als 60 % n-Pentyl-Reste sind.

4. Citronensäureester nach zumindest einem der Ansprüche 1 bis 3,
**dadurch gekennzeichnet,**
**dass** die Alkylreste von 70 bis 99,9 % n-Pentyl-Reste und von 30 bis 0,1 % Methylbutyl-Reste sind.

5. Citronensäureester gemäß Anspruch 4,
**dadurch gekennzeichnet,**
**dass** die Alkylreste von 85 bis 98 % n-Pentyl-Reste und von 15 bis 2 % Methylbutyl-Reste sind.

6. Citronensäureester nach Anspruch 4 oder 5,
**dadurch gekennzeichnet,**
**dass** die Methylbutyl-Reste vorzugsweise 2-Methylbutylreste sind.

7. Citronensäureester nach Anspruch 1 oder 2,
**dadurch gekennzeichnet,**
**dass** die Alkylreste R¹, R² und R³ zu mindestens 40 % 3-Methylbutylreste sind.

8. Citronensäureester nach Anspruch 7,
**dadurch gekennzeichnet,**
**dass** die Alkylreste R¹, R² und R³ zu 40 bis 100 %, insbesondere 50 bis 99 % 3-Methylbutylreste sind.

9. Citronensäureester nach einem der Ansprüche 1 bis 8,
**dadurch gekennzeichnet,**
**dass** der Rest R⁴ ein Acetylrest ist.

10. Verwendung von Citronensäureester der Formel I 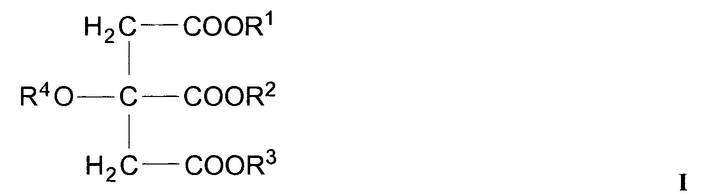 bei dem die Reste R¹, R² und R³ jeweils ein Alkylrest mit einer Anzahl an Kohlenstoffatomen von 5 sind und der Rest R⁴ ein Ameisensäurerest, ein Essigsäurerest, ein Propionsäurerest, ein Buttersäurerest oder ein Valeriansäurerest ist, als Weichmacher.

11. Verwendung von Citronensäureester der Formel I, worin
die Alkylreste R¹, R² und R³ eine längste Kohlenstoffkette von zumindest 4 Kohlenstoffatomen aufweisen und eine Gesamtzahl an Kohlenstoffatomen pro Alkylrest von 5 aufweisen, als Weichmacher.

12. Verwendung von Citronensäureester der Formel I, worin
die Alkylreste R¹, R² und R³ zu mehr als 60 % n-Pentyl-Reste sind, als Weichmacher.

13. Verwendung von Citronensäureester der Formel I, worin
die Alkylreste von 70 bis 99,9 % n-Pentyl-Reste und von 30 bis 0,1 % Methylbutyl-Reste sind, als Weichmacher.

14. Verwendung von Citronensäureester der Formel I, worin die Alkylreste von 85 bis 98 % n-Pentyl-Reste und von 15 bis 2 % Methylbutyl-Reste sind, als Weichmacher.

15. Verwendung von Citronensäureester der Formel I, worin die
Methylbutyl-Reste vorzugsweise 2-Methylbutylreste sind, als Weichmacher.

16. Verwendung von Citronensäureester der Formel I, worin die Alkylreste R¹, R² und R³ zu mindestens 40 % 3-Methylbutylreste sind, als Weichmacher.

17. Verwendung von Citronensäureester der Formel I, worin die Alkylreste R¹, R² und R³ zu 40 bis 100 %, insbesondere 50 bis 99 % 3-Methylbutylreste sind, als Weichmacher.

18. Verwendung nach den Ansprüchen 10 bis 17,
**dadurch gekennzeichnet,**
**dass** der Citronensäureester in Kunststoffzusammensetzungen, Klebstoffen, Dichtungsmassen, Lacken, Farben, Plastisolen, Kunstledern, Fußbodenbelägen, Unterbodenschutz, beschichteten Geweben, Tapeten, Tinten, Profilen, Dichtungen, Lebensmittelverpackungen, Folien, Spielzeug, Medizinalartikeln, Dachbahnen, Kabeln oder Drahtummantelungen als Weichmacher verwendet wird.

19. Zusammensetzung enthaltend einen Citronensäureester der Formel I und ein Polymer, ausgewählt aus Polyvinylchlorid (PVC), Polyvinylidenchlorid (PVDC), Polyacrylaten, Fluorpolymeren, Polyvinylacetat (PVAc), Polyvinylalcohol (PVA), Polyvinylacetale, Polystyrolpolymere, Polyolefine, Thermoplastische Polyolefine (TPO), Polyethylen-Vinylacetat (EVA), Polycarbonate, Polyethylenterephthalat (PET), Polybutylenterephthalat (PBT), Polyoxymethylen (POM), Polyamid (PA), Polyethylenglykol (PEG), Polyurethan (PU), Thermoplastisches Polyurethan (TPU), Polysulfide (PSu), Biopolymere, Polyester, Stärke, Cellulose und Cellulose-Derivate, Gummi, Silikone sowie Mischungen oder Copolymere der genannten Polymere oder deren monomeren Einheiten.

20. Zusammensetzung nach Anspruch 19,
**dadurch gekennzeichnet,**
**dass** sie die Citronensäureester im Gemisch mit anderen Weichmachern ausgewählt aus der Gruppe der Phthalsäuredialkylester, der Trimellitsäuretrialkylester, der Adipinsäuredialkylester, der Terephthalasäuredialkylester, der 1,2-Cyclohexandisäurealkylester, der 1,3-Cyclohexandisäurealkylester, der 1,4-Cyclohexandisäurealkylestern, der Dibenzoesäureester von Glykolen, der Alkylsulfonsäurester von Phenol, der Polymerweichmacher, der Glycerinester, der Citronensäuretrialkylester mit freier oder carboxylierter OH-Gruppe und einem Alkylrest von 4 oder 6 bis 10 C-Atomen und der Benzoesäurealkylester aufweisen.

21. Zusammensetzung nach Anspruch 20,
**dadurch gekennzeichnet,**
**dass** der Anteil an Citronensäureestern in diesem Gemisch 15 bis 90 Massen-% beträgt, wobei sich die Massenanteile aller vorhandenen Weichmacher zu 100 % addieren.

22. Zusammensetzung nach zumindest einem der Ansprüche 19 bis 21,
**dadurch gekennzeichnet,**
**dass** sie zumindest einen Benzoesäurealkylester mit Alkyl = Alkylrest mit 7 bis 13 Kohlenstoffatomen aufweist.

23. Zusammensetzung gemäß Anspruch 22,
**dadurch gekennzeichnet,**
**dass** Benzoesäurealkylester Benzoesäureisononylester, Benzoesäurenonylester, Benzoesäureisodecylester oder Benzoesäuredecylester ist.

24. Zusammensetzung nach Anspruch 19,
**dadurch gekennzeichnet,**
**dass** sie als PVC-Typ Suspensions-, Masse-, Mikrosuspensions- oder Emulsions-PVC enthält.

25. Zusammensetzung gemäß zumindest einem der Ansprüche 19 bis 24,
**dadurch gekennzeichnet,**
**dass** sie weitere Weichmacher, Füllstoffe, Pigmente, Stabilisatoren, Gleitmittel, Treibmittel, Kicker, Antioxidanzien oder Biozide enthält.

26. Zusammensetzung gemäß zumindest einem der Ansprüche 19 bis 25,
**dadurch gekennzeichnet,**
**dass** die Zusammensetzung eine Kunststoffzusammensetzung, ein Klebstoff, eine Dichtungsmasse, ein Lack, eine Farbe, ein Plastisol, ein Kunstleder, ein Fußbodenbelag, ein Unterbodenschutz, ein beschichtetes Gewebe, eine Tapete, eine Tinte, ein Profil, eine Dichtung, eine Lebensmittelverpackung, eine Folie, ein Spielzeug, ein Medizinalartikel, eine Dachbahn, ein Kabel oder eine Drahtummantelung ist oder als solche verwendet wird.

## Claims

1. Citric ester of the formula I, 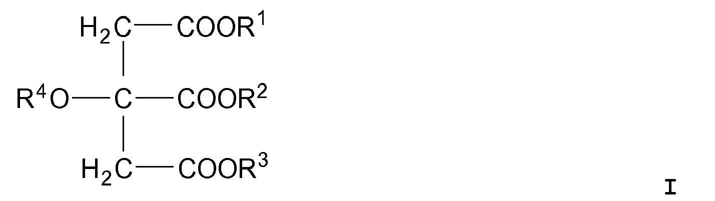 **characterized in that**
each of the radicals R¹, R² and R³ is an alkyl radical whose number of carbon atoms is 5 and the radical R⁴ is a formic acid radical, an acetic acid radical, a propionic acid radical, a butyric acid radical or a valeric acid radical.

2. Citric ester according to Claim 1,
**characterized in that**
the alkyl radicals R¹, R² and R³ have a longest carbon chain of at least 4 carbon atoms and their total number of carbon atoms per alkyl radical is 5.

3. Citric ester according to Claim 1 or 2,
**characterized in that**
more than 60% of the alkyl radicals R¹, R² and R³ are n-pentyl radicals.

4. Citric ester according to at least one of Claims 1 to 3,
**characterized in that**
from 70 to 99.9% of the alkyl radicals are n-pentyl radicals and from 30 to 0.1% of the alkyl radicals are methylbutyl radicals.

5. Citric ester according to Claim 4,
**characterized in that**
from 85 to 98% of the alkyl radicals are n-pentyl radicals and from 15 to 2% of the alkyl radicals are methylbutyl radicals.

6. Citric ester according to Claim 4 or 5,
**characterized in that**
the methylbutyl radicals are preferably 2-methylbutyl radicals.

7. Citric ester according to Claim 1 or 2,
**characterized in that**
at least 40% of the alkyl radicals R¹, R² and R³ are 3-methylbutyl radicals.

8. Citric ester according to Claim 7,
**characterized in that**
from 40 to 100%, in particular from 50 to 99%, of the alkyl radicals are 3-methylbutyl radicals.

9. Citric ester according to one of Claims 1 to 8,
**characterized in that**
the radical R⁴ an acetyl radical.

10. Use of a citric ester of the formula I 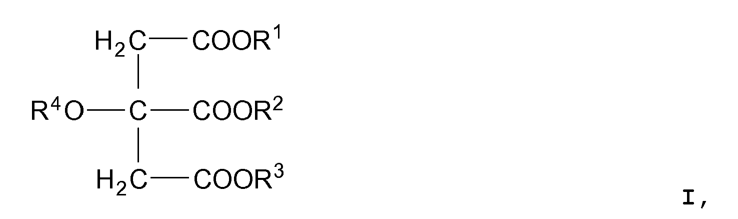 in which each of the radicals R¹, R² and R³ is an alkyl radical whose number of carbon atoms is 5 and the radical R⁴ is a formic acid radical, an acetic acid radical, a propionic acid radical, a butyric acid radical or a valeric acid radical, as plasticizer.

11. Use of a citric ester of the formula I in which the alkyl radicals R¹, R² and R³ have a longest carbon chain of at least 4 carbon atoms and their total number of carbon atoms per alkyl radical is 5, as plasticizer.

12. Use of a citric ester of the formula I in which more than 60% of the alkyl radicals R¹, R² and R³ are n-pentyl radicals, as plasticizer.

13. Use of a citric ester of the formula I in which from 70 to 99.9% of the alkyl radicals are n-pentyl radicals and from 30 to 0.1% of the alkyl radicals are methylbutyl radicals, as plasticizer.

14. Use of a citric ester of the formula I in which from 85 to 98% of the alkyl radicals are n-pentyl radicals and from 15 to 2% of the alkyl radicals are methylbutyl radicals, as plasticizer.

15. Use of a citric ester of the formula I in which the methylbutyl radicals are preferably 2-methylbutyl radicals, as plasticizer.

16. Use of a citric ester of the formula I in which at least 40% of the alkyl radicals R¹, R² and R³ are 3-methylbutyl radicals, as plasticizer.

17. Use of a citric ester of the formula I in which from 40 to 100%, in particular from 50 to 99%, of the alkyl radicals are 3-methylbutyl radicals, as plasticizer.

18. Use according to Claims 10 to 17,
**characterized in that**
the citric ester is used in a plastics composition, in an adhesive, in a sealing composition, in a coating, in a paint, in a plastisol, in a synthetic leather, in a floorcovering, in underbody protection, in a coated textile, in a wallpaper, in an ink, in a profile, in a gasket, in a food-or-drink packaging, in a foil, in a toy, in a medical item, in roof sheeting, in a cable or in wire sheathing, as plasticizer.

19. Composition comprising a citric ester of the formula I and
a polymer selected from polyvinyl chloride (PVC), polyvinylidene chloride (PVDC), polyacrylates, fluoropolymers, polyvinyl acetate (PVAc), polyvinyl alcohol (PVA), polyvinyl acetals, polystyrene polymers, polyolefins, thermoplastic polyolefins (TPO), polyethylene-vinyl acetate (EVA), polycarbonates, polyethylene terephthalate (PET), polybutylene terephthalate (PBT), polyoxymethylene (POM), polyamide (PA), polyethylene glycol (PEG), polyurethane (PU), thermoplastic polyurethane (TPU), polysulphides (PSu), biopolymers, polyesters, starch, cellulose and cellulose derivatives, rubber, silicones and mixtures or copolymers of the polymers mentioned or of their monomeric units.

20. Composition according to Claim 19,
**characterized in that**
it comprises the citric ester in a mixture with other plasticizers selected from the group of the dialkyl esters of phthalic acid, of the trialkyl esters of trimellitic acid, of the dialkyl esters of adipic acid, of the dialkyl esters of terephthalic acid, of the alkyl esters of 1,2-cyclohexanedioic acid, of the alkyl esters of 1,3-cyclohexanedioic acid, of the alkyl esters of 1,4-cyclohexanedioic acid, of the dibenzoic esters of glycols, of the alkylsulphonic esters of phenol, of the polymeric plasticizers, of the glycerol esters, of the trialkyl esters of citric acid having a free or carboxylated OH group and having an alkyl radical of 4 or from 6 to 10 carbon atoms, and of the alkyl esters of benzoic acid.

21. Composition according to Claim 20,
**characterized in that**
the proportion of citric esters in this mixture is from 15 to 90% by weight, where the proportions by weight of all of the plasticizers present give a total of 100%.

22. Composition according to at least one of Claims 19 to 21,
**characterized in that**
it comprises at least one alkyl ester of benzoic acid where alkyl = alkyl radical having from 7 to 13 carbon atoms.

23. Composition according to Claim 22,
**characterized in that**
alkyl ester of benzoic acid is isononyl benzoate, nonyl benzoate, isodecyl benzoate or decyl benzoate.

24. Composition according to Claim 19,
**characterized in that**
it comprises, as PVC grade, suspension PVC, bulk PVC, microsuspension PVC or emulsion PVC.

25. Composition according to at least one of Claims 19 to 24,
**characterized in that**
it comprises other plasticizers, fillers, pigments, stabilizers, lubricants, blowing agents, kickers, antioxidants or biocides.

26. Composition according to at least one of Claims 19 to 25,
**characterized in that** the composition is a plastics composition, an adhesive, a sealing composition, a coating, a paint, a plastisol, a synthetic leather, a floorcovering, underbody protection, a coated textile, a wallpaper, an ink, a profile, a gasket, a food-or-drink packaging, a foil, a toy, a medical item, roof sheeting, a cable or wire sheathing, or is used as such.

## Revendications

1. Ester d'acide citrique de formule I, 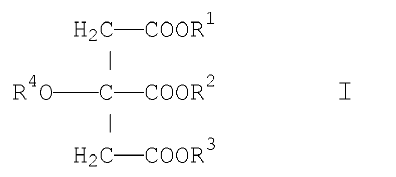 **caractérisé en ce que** les radicaux R¹, R² et R³ représentent chacun un radical alkyle ayant un nombre d'atomes de carbone égal à 5 et le radical R⁴ représente un reste d'acide formique, un reste d'acide acétique, un reste d'acide propionique, un reste d'acide butyrique ou un reste d'acide valérique.

2. Ester d'acide citrique selon la revendication 1, **caractérisé en ce que** les radicaux alkyle R¹, R² et R³ comportent une chaîne carbonée la plus longue d'au moins 4 atomes de carbone et un nombre total d'atomes de carbone par radical alkyle égal à 5.

3. Ester d'acide citrique selon la revendication 1 ou 2, **caractérisé en ce que** les radicaux alkyle R¹, R² et R³ sont à raison de plus de 60 % des radicaux n-pentyle.

4. Ester d'acide citrique selon au moins l'une des revendications 1 à 3, **caractérisé en ce que** les radicaux alkyle sont à raison de 70 à 99,9 % des radicaux n-pentyle et à raison de 30 à 0,1 % des radicaux méthylbutyle.

5. Ester d'acide citrique selon la revendication 4, **caractérisé en ce que** les radicaux alkyle sont à raison de 85 à 98 % des radicaux n-pentyle et à raison de 15 à 2 % des radicaux méthylbutyle.

6. Ester d'acide citrique selon la revendication 4 ou 5, **caractérisé en ce que** les radicaux méthylbutyle sont de préférence des radicaux 2-méthylbutyle.

7. Ester d'acide citrique selon la revendication 1 ou 2, **caractérisé en ce que** les radicaux alkyle R¹, R² et R³ sont à raison d'au moins 40 % des radicaux 3-méthylbutyle.

8. Ester d'acide citrique selon la revendication 7, **caractérisé en ce que** les radicaux alkyle R¹, R² et R³ sont à raison de 40 % à 100 %, en particulier de 50 à 99 % des radicaux 3-méthylbutyle.

9. Ester d'acide citrique selon l'une quelconque des revendications 1 à 8, **caractérisé en ce que** le radical R⁴ est un radical acétyle.

10. Utilisation d'ester d'acide citrique de formule I, 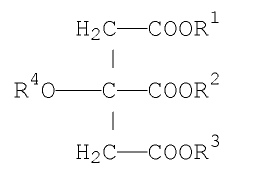 dans laquelle les radicaux R¹, R² et R³ représentent chacun un radical alkyle ayant un nombre d'atomes de carbone égal à 5 et le radical R⁴ représente un reste d'acide formique, un reste d'acide acétique, un reste d'acide propionique, un reste d'acide butyrique ou un reste d'acide valérique, en tant que plastifiant.

11. Utilisation d'ester d'acide citrique de formule I, dans lequel les radicaux alkyle R¹, R² et R³ comportent une chaîne carbonée la plus longue d'au moins 4 atomes de carbone et un nombre total d'atomes de carbone par radical alkyle égal à 5, en tant que plastifiant.

12. Utilisation d'ester d'acide citrique de formule I, dans lequel les radicaux alkyle R¹, R² et R³ sont à raison de plus de 60 % des radicaux n-pentyle, en tant que plastifiant.

13. Utilisation d'ester d'acide citrique de formule I, dans lequel les radicaux alkyle sont à raison de 70 à 99,9 % des radicaux n-pentyle et à raison de 30 à 0,1 % des radicaux méthylbutyle, en tant que plastifiant.

14. Utilisation d'ester d'acide citrique de formule I, dans lequel les radicaux alkyle sont à raison de 85 à 98 % des radicaux n-pentyle et à raison de 15 à 2 % des radicaux méthylbutyle, en tant que plastifiant.

15. Utilisation d'ester d'acide citrique de formule I, dans lequel les radicaux méthylbutyle sont de préférence des radicaux 2-méthylbutyle, en tant que plastifiant.

16. Utilisation d'ester d'acide citrique de formule I, dans lequel les radicaux alkyle R¹, R² et R³ sont à raison d'au moins 40 % des radicaux 3-méthylbutyle, en tant que plastifiant.

17. Utilisation d'ester d'acide citrique de formule I, dans lequel les radicaux alkyle R¹, R² et R³ sont à raison de 40 % à 100 %, en particulier de 50 à 99 % des radicaux 3-méthylbutyle, en tant que plastifiant.

18. Utilisation selon les revendications 10 à 17, **caractérisée en ce que** l'ester d'acide citrique est utilisé comme plastifiant dans des compositions de matières plastiques, des adhésifs, des matières d'étanchéité, des laques, des peintures, des Plastisols, des cuirs artificiels, des revêtements de sol, la protection de bas de caisse, des tissus enduits, des papiers peints, des encres, des profilés, des joints d'étanchéité, des emballages de produits alimentaires, des films, des jouets, des articles médicaux, des feuilles d'étanchéité pour toits, des câbles ou des revêtements de toitures.

19. Composition contenant un ester d'acide citrique de formule I et un polymère choisi parmi le poly(chlorure de vinyle) (PVC), le poly(chlorure de vinylidène) (PVDC), les polyacrylates, les polymères fluorés, le poly(acétate de vinyle) (PVAc), le poly(alcool vinylique) (PVA), les polyvinylacétals, les polymères polystyrène, les polyoléfines, les polyoléfines thermoplastiques (TPO), le poly(éthylène-acétate de vinyle) (EVA), les polycarbonates, le poly(éthylène-téréphtalate) (PET), le poly(butylène-téréphtalate) (PBT), le polyoxyméthylène (POM), le polyamide (PA), le polyéthylèneglycol (PEG), le polyuréthanne (PU), le polyuréthanne thermoplastique (TPU), les polysulfures (PSu), les biopolymères, les polyesters, l'amidon, la cellulose et les dérivés de cellulose, le caoutchouc vulcanisé, les silicones ainsi que des mélanges ou copolymères des polymères cités ou de leurs motifs monomères.

20. Composition selon la revendication 19, **caractérisée en ce qu'**elle contient les esters d'acide citrique en mélange avec d'autres plastifiants choisis dans le groupe des phtalates de dialkyle, des trimellitates de trialkyle, des adipates de dialkyle, des téréphtalates de dialkyle, des 1,2-cyclohexanedioates d'alkyle, des 1,3-cyclohexanedioates d'alkyle, des 1,4-cyclohexanedioates d'alkyle, des dibenzoates de glycols, des alkylsulfonates de phénol, des plastifiants polymères, des esters de glycérol, des citrates de trialkyle à groupe OH libre ou carboxylé et comportant un radical alkyle ayant 4 ou 6 à 10 atomes de carbone et des benzoates d'alkyle.

21. Composition selon la revendication 20, **caractérisée en ce que** la proportion d'esters d'acide citrique dans ce mélange vaut de 15 à 90 % en masse, la somme des proportions en masse de tous les plastifiants présents étant égale à 100 %.

22. Composition selon au moins l'une des revendications 19 à 21, **caractérisée en ce qu'**elle comporte au moins un benzoate d'alkyle où alkyle = radical alkyle ayant de 7 à 13 atomes de carbone.

23. Composition selon la revendication 22, **caractérisée en ce que** le benzoate d'alkyle est le benzoate d'isononyle, le benzoate de nonyle, le benzoate d'isodécyle ou le benzoate de décyle.

24. Composition selon la revendication 19, **caractérisée en ce qu'**elle contient en tant que type de PVC du PVC en suspension, en masse, en microsuspension ou en émulsion.

25. Composition selon au moins l'une des revendications 19 à 24, **caractérisée en ce qu'**elle contient d'autres plastifiants, des charges, des pigments, des stabilisants, des lubrifiants, des agents d'expansion, des amorceurs, des antioxydants ou des biocides.

26. Composition selon au moins l'une des revendications 19 à 25, **caractérisée en ce que** la composition est une composition de matière plastique, un adhésif, une matière d'étanchéité, une laque, une peinture, un Plastisol, un cuir artificiel, un revêtement de sol, une protection de bas de caisse, un tissu enduit, un papier peint, une encre, un profilé, un joint d'étanchéité, un emballage pour produit alimentaire, un film, un jouet, un article médical, une feuille d'étanchéité pour toits, un câble ou un revêtement de toiture ou est utilisée en tant que tels.
